# EUROPEAN PATENT SPECIFICATION

(11) **EP 2 016 420 B1**
(45) Date of publication and mention of the grant of the patent: **10.12.2014**
(21) Application number: 07732699.9
(22) Date of filing: 08.05.2007
(51) Int. Cl.: G01N 33/68, C07K 14/47

(54) **HlSTONES**
HISTONE
HISTONES

(30) Priority: 09.05.2006 GB 0609119
(43) Date of publication of application: 21.01.2009
(73) Proprietor: THE UNIVERSITY OF BIRMINGHAM, Birmingham B15 2TT (GB)
(72) Inventor: NIGHTINGALE Karl, Peter, Edgbaston Birmingham B15 2TT (GB)
(74) Representative: Ward, David Ian
(86) International application number: PCT/GB2007/001670
(87) International publication number: WO 2007/132177

(56) References cited:
- WO-A-02/077231
- WO-A-02/084249
- WO-A-03/072752
- WO-A2-2007/123976
- US-A1- 2003 003 516
- US-A1- 2005 054 118
- ROBYR D ET AL: "Genomewide histone acetylation microarrays" METHODS : A COMPANION TO METHODS IN ENZYMOLOGY, ACADEMIC PRESS INC., NEW YORK, NY, US, vol. 31, no. 1, September 2003 (2003-09), pages 83-89, XP004441575 ISSN: 1046-2023

## Description

The present invention relates to histones, and particularly to histone-specific antibodies, and to antibodies against histone modifications. The invention extends to apparatus for screening and/or identifying such antibodies, and to methods for manufacturing such apparatus. The invention also extends to methods for screening and characterising antibodies exhibiting specificity for histones and modifications thereof. The invention also extends to methods for screening and characterising the effects of histone modifications on the activity and binding of histone specific enzymes and binding proteins.

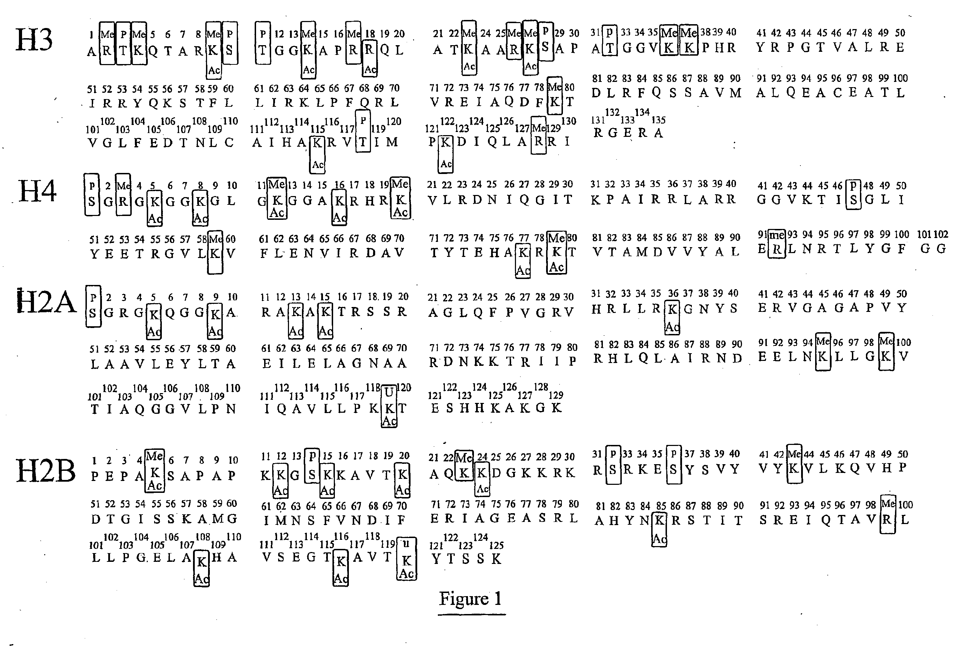

The histones are a class of proteins involved in the packaging of DNA, and are rich in arginine and/or lysine. The main classes of histones are designated H1 ('lysine-rich'), H2A and H2B ('slightly lysine-rich'), H3 and H4 ('arginine-rich'). Histones interact with DNA to form chromatin via salt bridges between positively charged arginine/lysine residues and the negatively-charged phosphate groups of DNA. Histones are involved in the regulation of all the molecular processes that need access to the DNA, such as gene expression and DNA replication. Hence, histones are involved in turning genes 'on' and 'off'. Histones appear to function by being marked with chemical marks or 'tags' (i.e. post translational modifications), which are attached to the histones. These tags are complicated for several reasons. Firstly, as shown in Figure 1, there are many types of chemical modifications that are deposited on the histones, such as acetylation, methylation, and phosphorylation, and secondly, they occur on many different residues within the histone proteins.

However, it is clear that what the modification is, and also where it is on the histone is important for regulating genes. Histone-mediated gene regulation is important for both the basic knowledge of biology, and also because it is important in many diseases when it goes wrong, such as in cancers, and leukaemias. Epigenetics, which is the field of study of the regulation of nuclear processes that does not require changes to the DNA sequence, and its associated therapies, aims to correct defects in gene expression by switching these genes back on, or off, as the case may be. Hence, epigenetics provides a new approach to the treatment of diseases, such as aging, inherited diseases and cancer.

Current research on histones and their various modifications is highly dependant on the availability and use of antibodies. These antibodies are ideally highly specific to a particular histone protein, a known histone modification, and also the modification site, for example, histone H3 serine10 phosphorylation. However, one problem with such histone antibodies is that they are very poorly characterised, and are often non-specific. For example, histone antibodies may recognise (i) phosphorylation of any amino acid residue, or (ii) serine phosphorylation, rather than (iii) serine phosphorylation at serine residue 10 of histone H3, i.e. in the context of AKS*^{P}*TG*.* A further problem with using histone antibodies is that adjacent modifications can also impact on antibody specificity. For example, H3 methylation at lysine 9 may influence recognition of serine 10 phosphorylation in histone H3.

Accordingly, the poor or incomplete definition of antibody specificity is a significant barrier to both academic and commercial research in the field of histones and their various modifications, such as in epigenetic analyses, because it is difficult to obtain antibodies, which exhibit high specificity to histone proteins or their modifications. Hence, there is a significant need in the field of histone research to provide apparatus and methods for detecting and isolating histone antibodies.

Other aspects of research relating to histones and their modifications is highly dependant on the availability and use of histone-specific enzymes, which catalyse the modification reactions to the amino acid residues on the histone proteins. Like antibodies, these enzymes are also highly specific to a particular histone protein, a known histone modification, and also the modification site, for example, histone H3 serine10 phosphorylation.

US 2005/0054118A1 discloses a high throughput screening method which employs a PVDF substrate for protein immobilization. One of the disclosed protein microarrays is described as an autoantigen array and includes histone proteins.

WO03/072752A2 is similar to US 2005/0054118A1. This document discloses a device for immbolization of proteins which includes a hydrophobic polymeric layer, preferably PVDF, attached to a rigid support. Methods of using the array are disclosed. One of the disclosed protein microarrays includes histone proteins and is used in a method of detecting an autoimmune disease.

US 2003/0003516A1 and WO02/084249 both disclose a method of determining the antibody specificity profile in an individual. The profile is determined through the binding or patient samples comprising antibodies, to arrays which can comprise antigens and epitopes. One of those arrays

includes histone, and is used to determine the autoantibody specificity in 8 different human autoimmune diseases.

WO02/077231 discloses human nucleic acid-associated proteins (NAAP) and polynucleotides which identify and encode NAAP. The document discloses an array of NAAP, or fragments of NAAP.

Robyr and Grunstein (2003) Methods 31, pages 83 to 89 Genomewide histone acetylation microarrays. This document discloses an approach to rapidly study histone modifications genomewide by combining chromatin immunoprecipitation and DNA microarrays.

WO2007/123976 discloses an array which comprises a variety of antigens for identifying auto antibodies in the assessment if responsiveness of therapy. The antigens may be post-translationally modified.

Hence, it is an object of the present invention to obviate or mitigate one or more of the problems of the prior art, whether identified herein or elsewhere, and to provide improved apparatus and methods for detecting, screening, identifying and/or isolating histone-specific antibodies, and antibodies showing specificity against the various specific histone modifications.

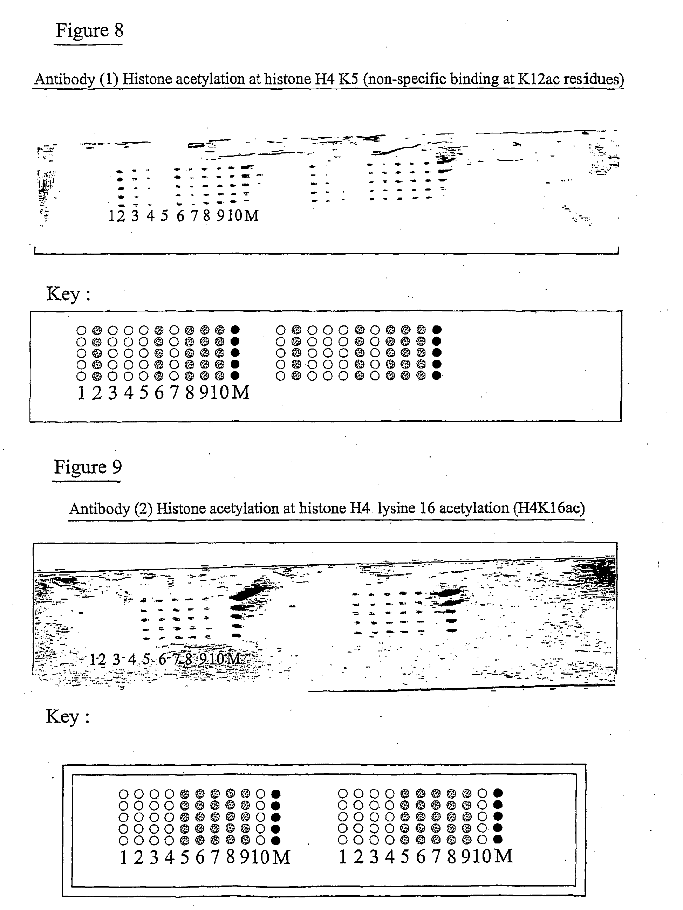

The inventors therefore set out to investigate the specificity of antibodies for various histone proteins (e.g. H3, H4, H2A and H2B) and their modifications, as summarised in Figure 1. They attached a series of peptides derived from histone proteins or modifications thereof, which acted as probe molecules, to a glass slide to form a histone microarray. The microarray was then exposed to solutions containing various antibodies, which were believed to have specificities for certain histones or histone modifications. As described in Examples 1 and 2, and as illustrated in Figures 8-13, the histone microarray was surprisingly useful for detecting, isolating and/or characterising antibodies having specificity not only for various histone proteins, histone isoforms, histone variants thereof, but surprisingly, also the post-translational modifications of such histone proteins.

Hence, according to a first aspect of the present invention, there is provided a histone microarray for characterising the specificity of histone-specific proteins, said microarray comprising a substrate support functionalised with a plurality of probe molecules, each probe molecule comprising a peptide sequence, or a derivative or analogue thereof, derived from a histone protein, or isoform, or variant thereof, or one of a series of post-translational modifications thereof.

Preferably, at least one probe molecule is adapted to bind to a histone immunoglobulin target molecule, such as a histone antibody target molecule. However, it is preferred that each probe molecule is adapted to bind to a histone antibody target molecule. Hence, the microarray according to the invention may be referred to as a histone antibody microarray, or screen. The inventors have demonstrated that, because the microarray according to the invention incorporates a plurality of probe molecules derived from a histone protein, or isoform, or variant, or modification thereof, the array enables a user to compare antibody binding between the probe molecules. Hence, the microarray provides a surprisingly effective, easy, and cheap method for detecting, screening, identifying, isolating, and/or characterising a histone antibody having specificity for a histone protein, and any isoforms, variants or post-translational modifications thereof.

Hence, according to a related aspect of the present invention, there is provided a method of detecting a histone antibody, the method comprising:-
(i) contacting a histone antibody target molecule with a histone microarray according to the first aspect of the invention; and
(ii) detecting the presence or absence of a histone antibody attached to a probe molecule.

Following the detection step (ii), the method preferably comprises a step of screening, identifying, isolating, and/or characterising the detected histone antibody. Hence, the invention provides both a histone microarray, and also a method for screening histone-specific, and modified histone specific antibodies, including histone variants and isoforms.

The peptide sequence in each probe molecule, which is derived from a histone protein, or isoform, or variant, or modification thereof, may be a derivative or analogue thereof.

By the term "derived from", we mean the peptide, derivative or analogue thereof comprises, or is a derivative or modification, of an amino acid sequence forming a histone protein, or isoform, or variant, or modification thereof. Hence, each probe molecule that is functionalised on the substrate support comprises a peptide, derivative or analogue thereof, which is based on the amino acid sequence forming a histone protein, or isoform, or variant, or modification thereof. Surprisingly, peptides, derivatives, or analogues which are derived from a histone protein, or isoform, or variant, or modification thereof, have been shown to act as functional probe molecules on the microarray and, in particular, have been shown to act as useful probes for detecting histone antibodies.

By the term "derivative or analogue thereof", we mean that the amino acid residues of the sequence derived from a histone protein, or isoform, or variant, or modification thereof, may be replaced by residues (whether natural amino acids, non-natural amino acids or amino acid mimics) with similar side chains or peptide backbone properties. Additionally, the terminals of such peptides may be protected by N- or C-terminal protecting groups with similar properties to acetyl or amide groups. It will also be appreciated that modified amino acids may be substituted into histone-derived peptides, or derivatives or analogues thereof with a number of amino acid variants that may be known to those skilled in the art to form further preferred derivatives or analogues according to the invention. Such derivative or analogue peptides will act as useful probe molecules provided that the modification does not significantly alter its chemical characteristics. For instance, hydrogens on the side chain amines of R or K may be replaced with methylene groups (-NH₂ → -NH(Me) or -N(Me)₂). Furthermore, the N-terminal amino group of the peptides may be protected by reacting with a carboxylic acid and the C-terminal carboxyl group of the peptide may be protected by reacting with an amine. A further embodiment of an analogue of a peptide used according to the invention comprises D-amino acid forms of the peptide.

In embodiments where the microarray is used to detect histone antibodies, the sequence that is derived from a histone protein, or isoform, or variant, or modification thereof, may be referred to as an epitope sequence. By the term "epitope sequence", we mean a sequence, which is recognisable and targeted by an antibody.

By the term "histone protein", we mean any one of the proteins, which form the major components of chromatin in most eukaryotes. The skilled technician will appreciate that there are various types of histone protein, such as H1, H2A, H2B, H3 and H4. Hence, it is preferred that the microarray according to the invention comprises at least two probe molecules comprising a sequence, which is derived from a histone protein independently selected from a group of proteins consisting of:- histone 1 (H1); histone 2A (H2A); histone 2B (H2B); histone 3 (H3); and histone 4 (H4). Hence, the microarray may comprise at least two probe molecules comprising a sequence derived from histone H1 protein, histone H2A protein, histone H2B protein, histone 3 protein, and/or histone 4 protein, or an isoform, or a variant, or a modification of any of such histone proteins.

Preferably, the microarray comprises probe molecules comprising sequences, which are derived from a human histone protein, or an isoform or variant or modification thereof. The protein sequences of these proteins are best defined by a unique accession number readily available on the 'Swissprot/Uniprot' database (http://www.ebi.uniprot.org/index.shtml). It will be appreciated that the histone proteins are amongst the most highly evolutionarily conserved proteins. Hence, most mammalian histone proteins are very similar and often identical to each other, i.e. human histone 1 is very similar to ape histone 1, for example. However, there tends to be some variation between the sequences of each histone protein, which variation results in the formation of histone isoforms and variants of each histone protein. Histone isoforms are highly conserved histone proteins, and occur for all of the 'standard' histone proteins (i.e. H1, H2A, H2B, H3 & H4). Histone isoforms differ from each other by containing small numbers (about 1-3) of amino acid substitutions. They are typically highly abundant proteins, but the functional reason for the existence of these different isoforms is unclear.

By way of example, the Swissprot accession numbers for the most abundant human histone 1 (H1) isoforms are H1.0 (P16401), H1.1 (Q02539), H1.2 (P16403), H1.3 (P16402), H1.4 (P10412) and H1.5 (P16401). The Swissprot accession numbers for human histone 2A (H2A) isoforms are H2A.1 (POCOS8), H2A.2 (Q6FI13), and H2A.3 (P20671). The Swissprot accession numbers for human histone 2B (H2B) isoforms are H2A.a (P62807), H2A.b (P58876), H2A.c (Q99880), H2A.d (Q99877), H2A.e (QQ99879) and H2A.f (P33778). The Swissprot accession numbers for human histone H3 (H3) isoforms are H3.1 (P68431) and H3.3 (P84243). The Swissprot accession number for human histone H4 (H4) is P62805.

Hence, by the term "histone isoform", we mean a modified version of a histone protein (i.e. H1, H2A, H2B, H3 or H4) comprising small differences in amino acid sequence. Hence, a histone isoform may comprise at least about 85% identity with the histone protein to which it corresponds (e.g. human H3.1 with human H3.3).

Accordingly, preferred histone isoforms, which may be used as probe molecules, may be independently selected from a group of histone isoforms consisting of:- H1.0; H1.1; H1.2; H1.3; H1.4; H1.5; H2A.1; H2A.2; H2A.3; H2B.a; H2B.b; H2B.c, H2A.d, H2A.e, H2A.f; H3.1; H3.2; H3.3; and H4, and their related homologues between species.

In addition to histone isoforms, histone variants also occur for several of the standard histones (for example, H3 & H2A). Histone variants consist of at least two distinct regions: (i) a highly conserved 'core' region (or domain), which is substantially identical to the corresponding region of a standard histone sequence (for example H3); and (ii) one or two variable regions at the N- or C-terminal 'tails' of the protein which are highly divergent from the corresponding region in the standard histone sequence. Current data suggests that histone variants are involved in the packaging of DNA. However, the variant protein sequence(s) bring different functional characteristics. Histone variants are associated with functionally distinct regions of the chromosomes.

By way of example, human histone variants may include H2A-X (Swissprot Acc. No. P16104), H2A-Z (Swissprot Acc. No. POCKS5), CENP-A (Swissprot Acc. No. P49450). CENP-A is found on the centromeres.

Hence, by the term "histone variant", we mean a modified version of a histone protein comprising larger differences in amino acid sequence than that of histone isoforms, for example, comprising less than about 85% identity with the histone protein to which it corresponds (e.g. human CENP-A and H3.1).

Accordingly, preferred histone variants, which may be used in the probe molecules, may be independently selected from a group of variants consisting of: H2A-X; H2A-Z; macro-H2A, H2A-Bbd, CENP-A; and their related homologues in other species.

It will also be appreciated that histone proteins or isoforms or variants thereof may each be chemically modified with chemical 'marks' or 'tags', which-are attached to certain amino acid residues thereof. These tags or marks may occur on many different parts of the histone protein or isoform or variant thereof, and may consist of acetylation, methylation, ubiquitination, and phosphorylation. As illustrated in Figure 1, by way of example, histone modifications may include lysine methylation, lysine acetylation, lysine ubiquitination, serine phosphorylation, threonine phosphorylation and arginine methylation.

Hence, by the term "histone modification", we mean the post-translational chemical modification, marking or tagging of at least one amino acid residue in the histone protein, or isoform or variant thereof. Preferred histone modifications, which may be used as sequences in the probe molecules may be independently selected from: - histone phosphorylation; methylation; acetylation; and ubiquitination. Especially preferred histone modifications, which may be used as sequences in the probe molecules may be independently selected from:- methylation of arginine or lysine; phosphorylation of threonine or serine; acetylation of lysine; or ubiquitination of lysine. Furthermore, it will be appreciated that lysine may be mono-, di-, or tri-methylated, and that arginine may be mono- or di-methylated, which may also form preferred histone modifications.

It will be appreciated that the sequence of the probe molecules used in microarray will depend on the nature and sequence of the antibody target molecule to which they are intended to hybridise or screen. Accordingly, the skilled technician will appreciate that the invention should not be limited to any specific peptide sequences providing they are derived from a histone protein, or isoform, or variant, or a modification thereof. However, it is preferred that the peptide sequence comprises at least 10 amino acids, and more preferably, at least 15 amino acid residues in length. The skilled technician will appreciate that short (e.g. 10 amino acids or less) peptide sequences offer limited selectivity and sensitivity, whereas longer (e.g. 10 amino acids or more) peptide or polypeptide sequences offer improved selectivity and sensitivity, and still appreciably longer (e.g. 15 amino acids or more) peptide sequences offer further improved sensitivity. Hence, more preferably, the peptide sequences comprise at least 17 amino acids residues, still more preferably, at least 20 amino acids residues. Preferably, the peptide sequence comprises less than 100 amino acids, more preferably, less than 50 amino acid residues, and most preferably, less than 35 amino acids in length.

In one embodiment, the microarray may be functionalised with probe molecules comprising sequences that are derived from different histone proteins. For example, in one embodiment, at least one probe molecule on the microarray may comprise a sequence derived from histone H3, and at least one probe molecule may comprise a sequence derived from histone H4. In another embodiment, at least one probe molecule on the microarray may comprise a sequence derived from histone H1, and at least one probe molecule may comprise a sequence derived from histone H2A. The skilled technician will appreciate that many different combinations of histone derived sequences would be available. By using probe molecules derived from different histones, it is possible to carry out comparison experiments between the two histones chosen.

However, in a preferred embodiment, the microarray is functionalised with probe molecules comprising sequences that are derived from the same histone protein, for example, either histone H1, or histone H3, or histone H4, or histone H2A and/or histone H2B, thereby forming a histone H1, H3, H4, H2A, or H2B microarray, respectively.

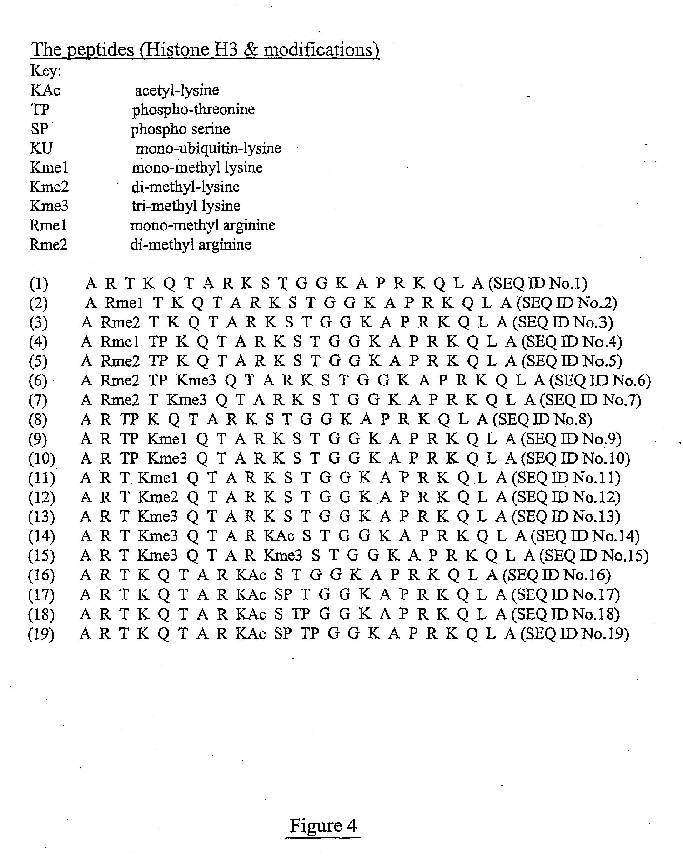

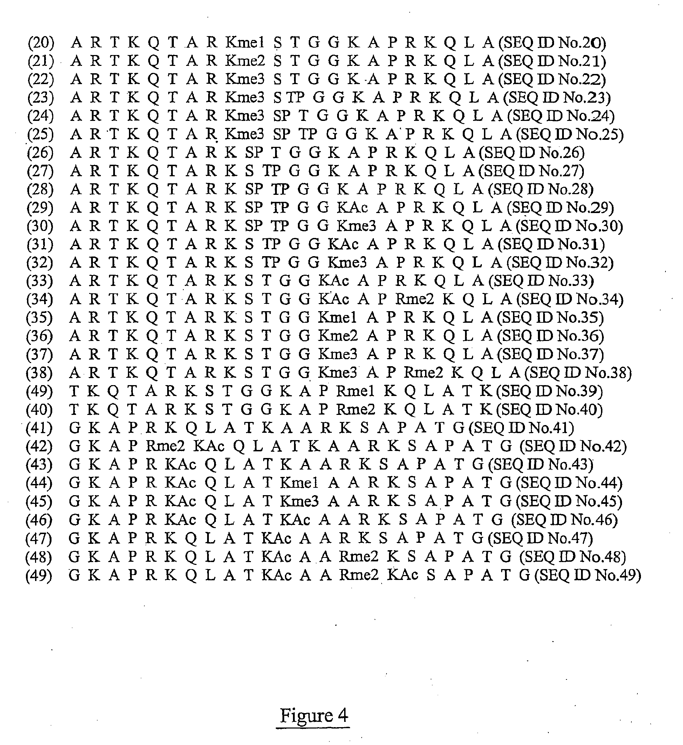

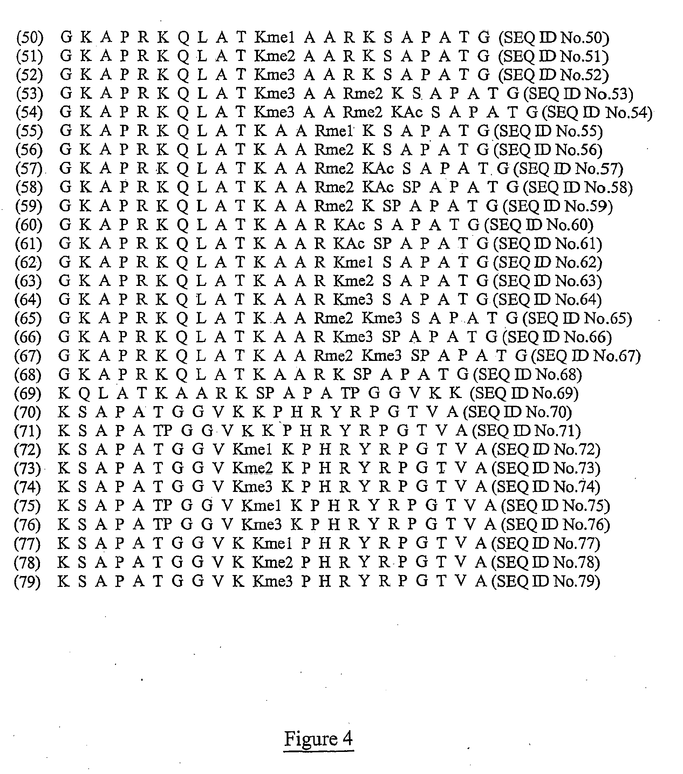

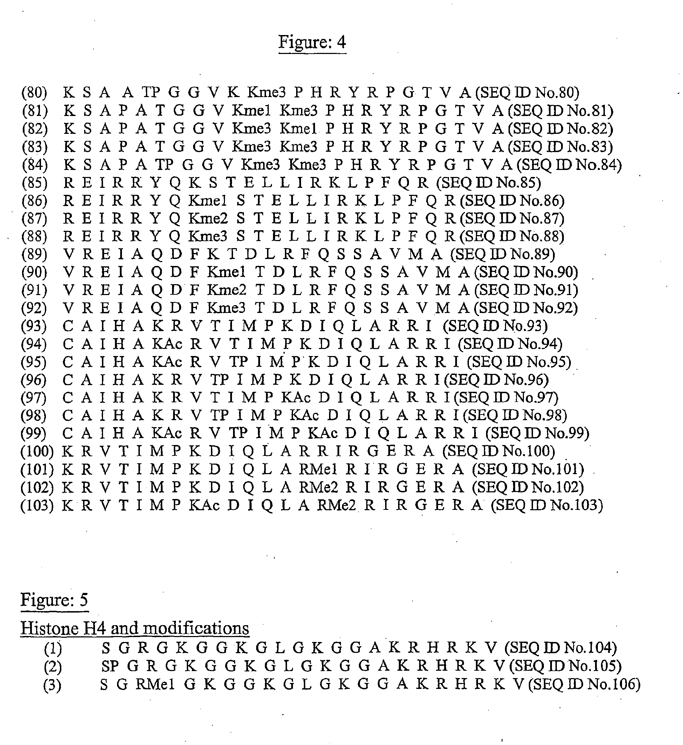

Hence, in one preferred embodiment, the microarray may comprise at least two probe molecules comprising a sequence derived from histone H3 protein, or isoform, or variant, or modification thereof. For example, Figure 4 shows a selection of suitable sequences derived from human histone H3 and modifications thereof, and which are referred to herein as SEQ ID No.1 to SEQ ID No. 103. The sequence identified as SEQ ID No.1 is a 21-mer of part of the human histone H3 protein. The sequence identified as SEQ ID No.2 is identical to SEQ ID No.1 except that the first arginine residue (R) is mono-methylated. The sequence identified as SEQ ID No.3 is identical to SEQ ID No.1 except that the first arginine residue (R) is di-methylated. The sequence identified as SEQ ID No.4 is identical to SEQ ID No.1 except that the first arginine residue (R) is monomethylated, and also the first threonine (T) residue is phosphorylated, and so on. The sequences identified as SEQ ID No's 39-103 are derived from a different region of histone H3 protein either with or without the various modifications.

Therefore, a preferred microarray may comprise at least two probe molecules comprising a sequence independently selected from a group of sequences identified as SEQ ID No.1 to SEQ ID No.103, or any combination thereof. Preferably, the microarray comprises a plurality of probe molecules comprising substantially all of the sequences identified as SEQ ID No.1 to SEQ ID No.103.

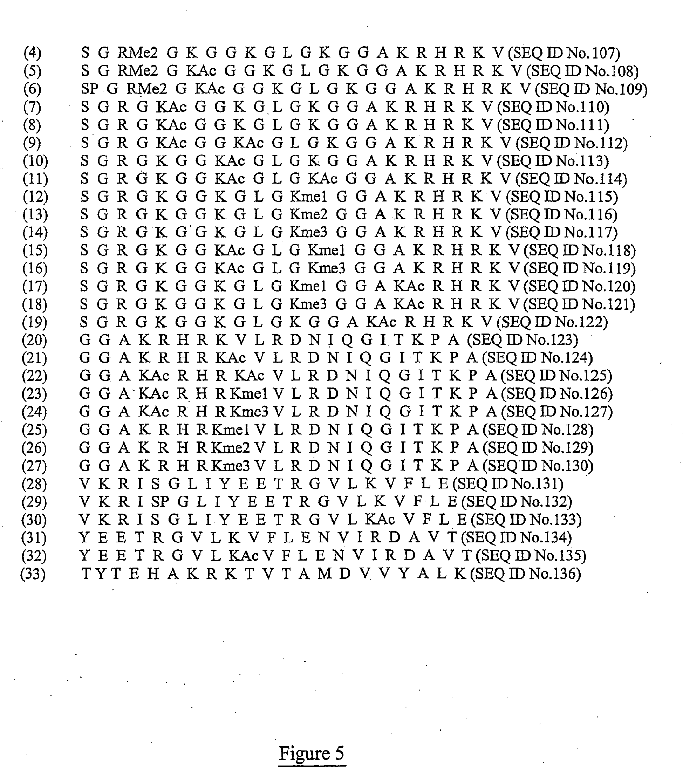

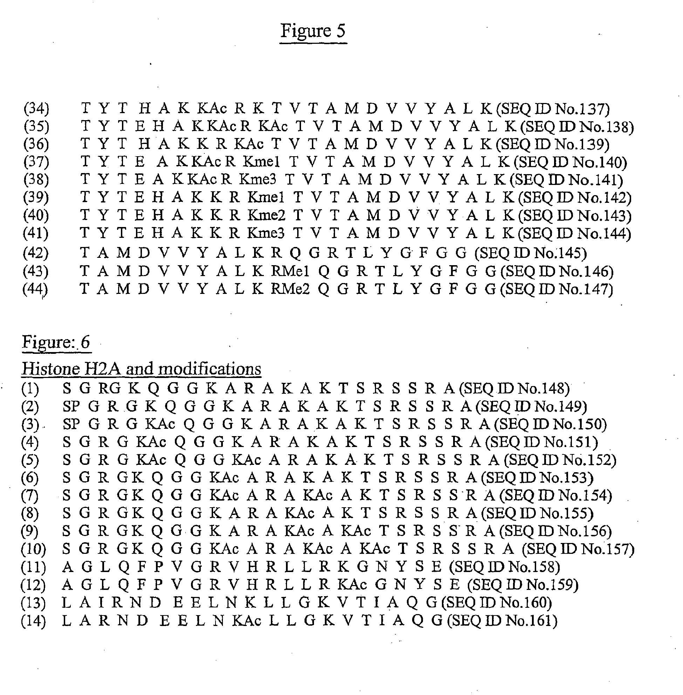

In a further preferred embodiment, the microarray may comprise at least two probe molecules comprising a sequence derived from histone H4 protein, or isoform, or variant, or modification thereof. For example, Figure 5 shows a selection of suitable sequences derived from human histone H4 and modifications thereof, and which are referred to herein as SEQ ID No. 104 to SEQ ID No. 147. The sequence identified as SEQ ID No.104 is a 21-mer of part of the human histone H4 protein. The sequence identified as SEQ ID No. 105 is identical to SEQ ID No. 104 except that the first serine residue (S) is phosphorylated. The sequence identified as SEQ ID No.106 is identical to SEQ ID No.104 except that the first arginine residue (R) is mono-methylated. The sequence identified as SEQ ID No. 107 is identical to SEQ ID No. 104 except that the first arginine residue (R) is di-methylated, and so on. The sequences identified as SEQ ID No's 123-147 are derived from a different region of histone H4 protein either with or without the various modifications.

Therefore, a preferred microarray may comprise at least two probe molecules comprising a sequence independently selected from a group of sequences identified as SEQ ID No.104 to SEQ ID No.147, or any combination thereof. Preferably, the microarray comprises a plurality of probe molecules comprising substantially all of the sequences identified as SEQ ID No.104 to SEQ ID No. 147.

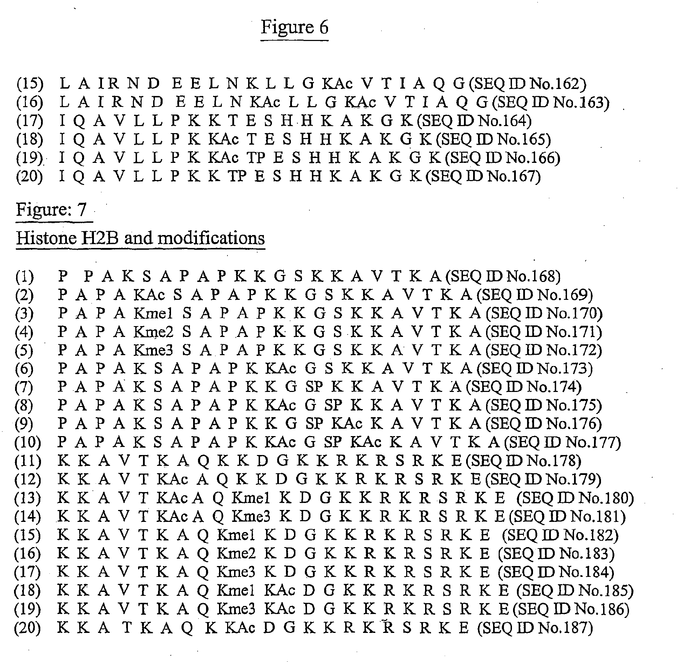

In a still further preferred embodiment, the microarray may comprise at least two probe molecules comprising a sequence derived from histone H2A protein, or isoform, or variant, or modification thereof. For example, Figure 6 shows a selection of suitable sequences derived from human histone H2A and modifications thereof, and which are referred to herein as SEQ ID No. 148 to SEQ ID No.167. The sequence identified as SEQ ID No. 148 is a 23-mer of part of the human histone H2A protein. The sequence identified as SEQ ID No.149 is identical to SEQ ID No.148 except that the first serine residue (S) is phosphorylated. The sequence identified as SEQ ID No.150 is identical to SEQ ID No.148 except that the first serine residue (S) is phosphorylated, and first lysine residue (K) is acetylated. The sequence identified as SEQ ID No.151 is identical to SEQ ID No.148 except that the first lysine residue (K) is acetylated, and so on. The sequences identified as SEQ ID No's 158-167 are derived from a different region of histone H2A protein either with or without the various modifications.

Therefore, a preferred microarray may comprise at least two probe molecules comprising a sequence independently selected from a group of sequences identified as SEQ ID No.148 to SEQ ID No.167, or any combination thereof. Preferably, the microarray comprises a plurality of probe molecules comprising substantially all of the sequences identified as SEQ ID No. 148 to SEQ ID No.167.

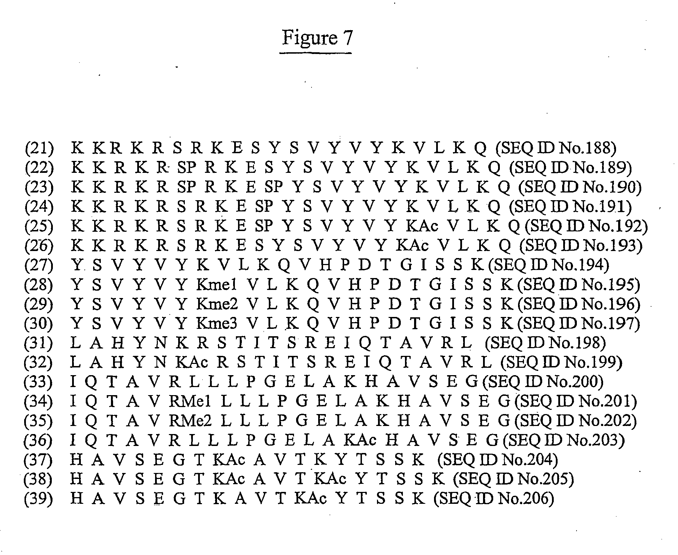

In a still further preferred embodiment, the microarray may comprise at least two probe molecules comprising a sequence derived from histone H2B protein, or isoform, or variant, or modification thereof. For example, Figure 7 shows a selection of suitable sequences derived from human histone H2B and modifications thereof, and which are referred to herein as SEQ ID No.168 to SEQ ID No.206. The sequence identified as SEQ ID No.168 is a 21-mer of part of the human histone H2B protein. The sequence identified as SEQ ID No.169 is identical to SEQ ID No.168 except that the first lysine residue (K) is acetylated. The sequence identified as SEQ ID No.170 is identical to SEQ ID No.168 except that the first lysine residue (K) is mono-methylated. The sequence identified as SEQ ID No.171 is identical to SEQ ID No.168 except that the first lysine residue (K) is di-methylated, and so on. The sequences identified as SEQ ID No's 178-206 are derived from a different region of histone H2B protein either with or without the various modifications.

Therefore, a preferred microarray may comprise at least two probe molecules comprising a sequence independently selected from a group of sequences identified as SEQ ID No.168 to SEQ ID No.206, or any combination thereof. Preferably, the microarray comprises a plurality of probe molecules comprising substantially all of the sequences identified as SEQ ID No. 168 to SEQ ID No.206.

It will be appreciated that due to the similarity between histones H2A and H2B, it is envisaged that it may be advantageous to have a microarray comprising a plurality of probe molecules comprising sequences derived from histone H2A protein and histone H2B protein, or an isoform, or variant, or modification thereof. Hence, in such an embodiment, the microarray may comprise at least two probe molecules comprising a sequence independently selected from a group of sequences identified as SEQ ID No. 148 to SEQ ID No.206, or any combination thereof. Preferably, the microarray comprises a plurality of probe molecules comprising substantially all of the sequences identified as SEQ ID No. 148 to SEQ ID No.206.

In a yet further preferred embodiment, the microarray may comprise at least two probe molecules comprising a sequence derived from histone H1 protein, or isoform, or variant, or modification thereof. From the foregoing, and from using publicly available databases, the skilled technician will appreciate the sequences of various histone H1 sequences peptides, which may be used as probe molecules, to form an H1 histone microarray. The Swissprot Accession Number for the most abundant human histone H1 isoforms are H1.0 (P16401), H1.1 (Q02539), H1.2 (P16403), H1.3 (P16402), H1.4 (P10412) and H1.5 (P16401).

The microarray may be histone specific (e.g. probes derived from H1 only, or H3 only etc), or the microarray may comprise probe molecules derived from all histones. It is therefore envisaged that the microarray according to the invention may be functionalised with probe sequences comprising sequences having any combination of any of the sequences identified as SEQ ID No.1 to SEQ ID No.206. Hence, it will be appreciated that the invention therefore provides a series of various microarrays, which are functionalised with probe molecules comprising sequences that are derived from either just one type of histone protein, or alternatively, various types of histone protein, and which can therefore be used for example as a histone H1 microarray, or a histone H3 microarray, and so on, to detect and subsequently characterise H1 or H3 specific antibodies using the method described above.

In one embodiment of the invention, the microarray according to the invention may comprise probe molecules comprising sequences derived from a histone protein, or isoform, or variant, or modification thereof, wherein at least two sequences are substantially the same sequence as each other, but which comprise at least one histone modification. Preferably, the modifications are different. By way of example only, the sequences identified as SEQ ID No.2-38 are identical to SEQ ID NO.1 (histone H3), but have at least one histone modification. It will be appreciated that some of the sequences have at least two histone modifications. The same applies to histones H2A, H2B, H1 and H4.

However, in another embodiment, the microarray may comprise probe molecules comprising sequences derived from a histone protein, or isoform, or variant, or modification thereof, wherein at least two sequences are substantially different from each other, and which may or may not comprise different histone modifications. By way of example only, the sequences identified as SEQ ID No.39-103 are different to SEQ ID NO.1 (H3), with some having at least one histone modification, and others having no histone modification. The same applies to histones H2A, H2B, H1 and H4.

The inventors set out to produce a microarray that was functionalised with a plurality of probe molecules forming a comprehensive series of sequences that reflected what is observed in living cells. Hence, it is preferred that the microarray comprises a substrate support functionalised with a plurality of probe molecules, each comprising a peptide sequence derived from a histone protein sequence containing:- (i) no histone modifications at all; (ii) at least one histone modification; and/or (ii) a plurality of histone modifications.

Hence, advantageously, the microarray according to the invention contains a systematic population of peptides, which when analysed together defines the specificity of antibodies, which bind to them. Furthermore, the method according to the invention is based on the use of a comprehensive and systematic array of unmodified and modified histone probe molecules. Therefore, advantageously, the inventors believe that the microarray and the method according to the invention may be effectively used for screening and/or identifying (i) histone-specific antibodies; (ii) antibodies against specific histone modifications, by exposing the microarray with a solution of antibody target molecules; and (iii) antibodies, which recognise histone modifications only in certain contexts (i.e. when adjacent residues that are unmodified). The invention therefore provides a systematic approach to allow a comprehensive and highly accurate definition of histone antibody specificity.

Hence, in a most preferred embodiment, the microarray comprises a substrate support functionalised with a plurality of probe molecules, each comprising a peptide sequence or a derivative or analogue thereof, derived from a histone protein, or isoform, or variant, or one of a series of post-translational modifications thereof, wherein at least one sequence comprises one or more modified amino acids, and wherein at least one other sequence comprises no modified amino acids. The inventors have found that this arrangement of probe molecules provides the ability to compare antibody binding to two or more slightly different sequences.

It will be appreciated that a substantial number of probe molecules on the microarray comprise a sequence derived from a histone protein, or isoform, or variant, or modification thereof. Therefore, suitably, the microarray according to the invention may comprise at least 3 probe molecules, more suitably, at least 4 probe molecules, and even more suitably, at least 5 probe molecules, each comprising a sequence derived from a histone protein, or isoform, or variant, or modification thereof. It is envisaged that the microarray comprises at least 10 probe molecules, more suitably, at least 20 probe molecules, and even more suitably, at least 30 probe molecules, each comprising a sequence derived from a histone protein, or isoform, or variant, or modification thereof. Preferably, the microarray comprises at least 40 probe molecules, more preferably, at least 50 probe molecules, and even more preferably, at least 60 probe molecules, each comprising a sequence derived from a histone protein, or isoform, or variant, or modification thereof. Still more preferably, the microarray comprises at least 80 probe molecules, more suitably, at least 100 probe molecules, and even more suitably, at least 120 probe molecules, each comprising a sequence derived from a histone protein, or isoform, or variant, or modification thereof.

In aspects related to the present invention, at least 10%, more suitably, at least 20%, even more suitably, at least 30%, and most suitably, at least 40% of the probe molecules comprise a sequence derived from a histone protein, or isoform, or variant, or modification thereof. However, it is preferred that at least 50%, more suitably, at least 60%, even more suitably, at least 70%, and most suitably, at least 80% of the probe molecules comprise a sequence derived from a histone protein, or isoform, or variant, or modification thereof. In most preferred embodiments, at least 85%, more suitably, at least 90%, even more suitably, at least 95%, and most suitably, at least 98% of the probe molecules comprise a sequence derived from a histone protein, or isoform, or variant, or modification thereof. In especially preferred embodiments, substantially all of the probe molecules comprise a sequence derived from a histone protein, or isoform, or variant, or modification thereof.

Therefore, in a most preferred embodiment the invention provides a microarray comprising peptide probe molecules as defined herein, which probe molecules collectively define all known histone modifications, and also their location in the various histone sequences. Hence, advantageously, the use of a systematic microarray according to the invention comprising probe molecules, which preferably, contains substantially all of the possible combinations of potential probes in cells will enable users to identify the three critical factors which define histone antibody specificity. Firstly, a user can identify exactly what class of chemical modification is recognised by the antibody (e.g. lysine can be acetylated, or mono-, di or tri-methylated). Secondly, a user may characterise the precise location(s) of the modification recognised by the antibody, i.e. the residue and it's surrounding sequence context. Thirdly, the user may also identify whether neighbouring histone modifications have an impact on antibody recognition.

Hence, upon detection of the histone antibody, the method enables the full characterisation of that antibody, i.e. (i) whether it is adapted to bind to a type of histone modification *per se* (e.g. phosphorylation, acetylation, or methylation etc); (ii) a type of histone modification on a type of amino acid residue (e.g. phosphorylation of serine residues); or (iii) a type of histone modification on a specific amino acid residue in the histone protein (e.g. phosphorylation of serine 10 on H3).

The inventors also believe that a method for preparing the histone microarray according to the first aspect will also have utility.

Alsoprovided is a method for preparing a histone microarray comprising a step of functionalising a substrate support with at least two probe molecules each comprising a sequence derived from a histone protein, or isoform, or variant, or modification thereof.

By the term "functionalise", we mean the attachment or coupling of the probe molecules to the substrate support. The term "spotting" may also be used to define the act of functionalising. The use of microarrays will be well-known to the skilled technician (For example, Schena, M., Microarray Biochip Technology, Eaton Publishing, Sunnyvale, CA, 87-187, 2000), as will the various types of substrate support which may be used in microarray technology. The substrate support may therefore comprise silicon, glass or polymer etc, or a nylon membrane, or a microtitre plate, which substrate is functionalised with the probe molecule.

Techniques for attaching or coupling a probe molecule to a support surface will also be known to the skilled technician, and may include use of electrostatic interaction between the probe molecule with a coating film of a polycationic polymer such as poly-L-lysine (as described in WO 95/35505) present on the support surface, or by using covalent bonding to the support by well-established techniques. Alternatively, substrates having metallic surfaces may be functionalised with a probe molecule by self-assembly that may involve a thiol linkage.

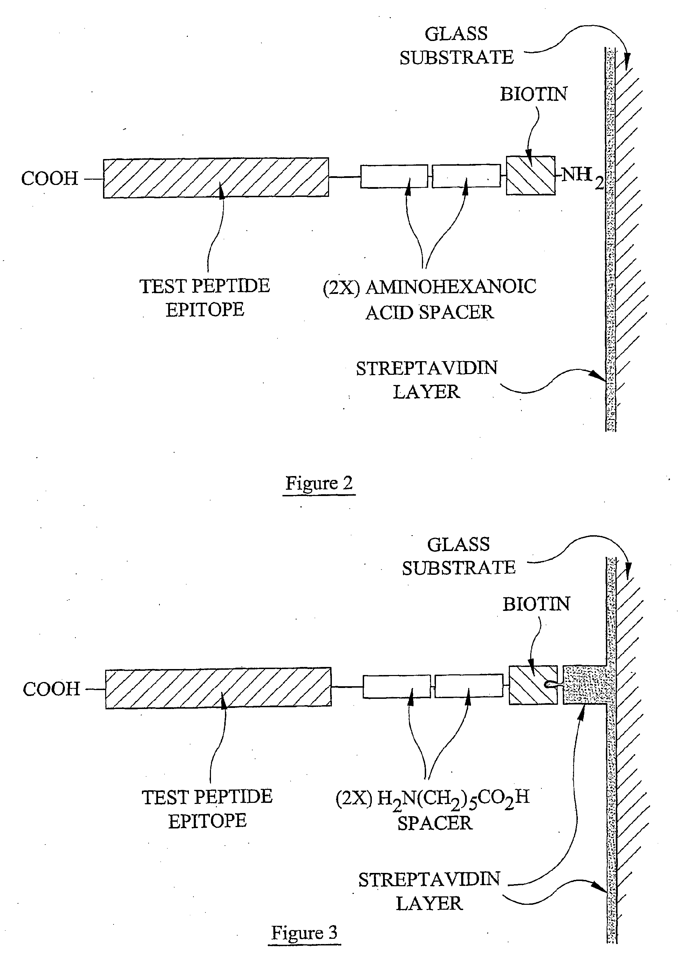

However, a preferred method comprises the use of covalent chemistry attachment methods by self-assembled monolayers, or by avidin or streptavidin to biotin associations. Hence, each probe molecule may comprise a biotin molecule, and the substrate may comprise streptavidin, and preferably a coating thereof. As shown in Figures 2 and 3, the substrate comprises a glass slide that was pre-coated with a layer of streptavidin, which irreversibly binds to biotin attached to the amino-teminus of the probe molecule thereby forming the micro-array.

During their experiments, the inventors found that the microarray was not always able to discriminate clearly between various antibodies if the (epitope) sequences derived from a histone protein were positioned too close to the surface of the substrate. They therefore carried out a series of experiments to determine the precise distance required between the epitope sequence and the substrate. They found that the microarray showed greatly improved specificity in embodiments where the epitope sequence was sufficiently spaced apart from the substrate surface. While the inventors do not wish to be bound by any hypothesis, they believe that the spacing enables the epitope sequences to protrude sufficiently far away from the substrate to avoid steric hindrance thereby allowing antibodies to hybridise with the probe molecules.

Accordingly, it is preferred that the probe molecule comprises spacing means, which spacing means is adapted to distance the sequence derived from a histone protein, isoform, variant or modification thereof, away from the substrate support when bound thereto. Preferably, the spacing means (or spacer) is disposed between the histone derived sequence and the site of attachment between the probe molecule and the substrate surface. Preferably, the spacing means is disposed between sequence and the biotin molecule in embodiments where biotin is used to attach the probe to the substrate.

The skilled technician will appreciate the types of chains, which may be incorporated in to the probe sequence as a spacing means or spacer. For example, the spacing means may comprise an alkyl chain, or an alkyl chain incorporating either amino, ester, amide or carbonyl groups, with appropriate functionalisation at it's termini for incorporation into the probe sequence. The incorporation of the spacing means in to the probe sequence may be, for example, by an ester or amide linkage.

The spacing means may comprise at least one, preferably at least two, and more preferably, at least three atoms in a chain. It will be appreciated that the actual type of atom is less important than the number and size of the atom, which impart the distancing effect of the epitope sequence away from the substrate support. Hence, by way of example, the atom(s) in the spacing means may be a carbon or oxygen atom, or combinations thereof. The spacing means may comprise a branched chain. However, preferably, the chain is straight. It is envisaged that the spacing means or spacer may comprise at least 5, 10, 15, 20, 25, or 30 or more atoms. It will be appreciated that the formula and length of the spacer will be determined by the type and length of the epitope sequence in the probe molecule.

The spacing means may comprise a plurality of interconnected amino acids. The skilled technician will appreciate how to interconnect amino acids to form a suitable spacing means for distancing the epitope sequence from the substrate support. By way of example, the spacing means may comprise beta Alanine (x3), GlySerGlySer or GlyGlyGlyGlyGly.

The spacing means may comprise a repeated unit, or chain. For example, the spacing means may comprise [-CH₂-]ₙ, wherein the value of n is an integer of at least 1. However, n is an integer, which may be greater than 1, and hence, is essentially a repeated unit of [-CH₂-]ₙ. Another example of a suitable spacing means comprises [-CH₂-O-CH₂-]ₙ, wherein n is an integer of at least one. However, n is an integer, which may be greater than 1, and hence, is essentially a repeated unit of CH₂-O-CH₂-]ₙ.

It is most preferred that the spacing means comprises at least one molecule of aminohexamoic acid (H₂N(CH₂)₅CO₂H). It will be appreciated that this constitutes a chain length of 7 atoms. However, as shown in Figures 2 and 3, the inventors have found that optimum spacing means comprises two molecules of aminohexamoic acid. Hence, it is preferred that the biotin residue of each test peptide is separated from the peptide sequence corresponding to the histone or histone modification by means of two contiguous aminohexanoic acid (H₂N(CH₂)₅CO₂H) spacer molecules. These spacers are included to move or space the test peptide sequence away from the immobilised portion of the molecule. Having just two aminohexanoic acid spacers allows quicker synthesis and a lower chance of the spacer being recognised by antibodies being screened with the microarray.

The microarray may be made by spotting a solution of each of probe molecule onto the substrate support in an appropriate grid-like formation, with each probe molecule being bound to the substrate as a monolayer. Hence, the probe molecules are preferably prepared in solution and diluted to an approximate concentration of about 5 nanomoles/ml, for example, in a solution of 60% acetonitrile, 40% water and 0.05% trifluoracetic acid. The inventors have found that that substantially higher concentrations of the probe molecule results in smearing during the detection step of the method, and hence, created problems of interpretation. Accordingly, it is preferred that probe molecule concentrations of less than 5nmole/ml are used to functionalise the substrate.

In a preferred embodiment, the microarray may comprise a library or population of probe molecules arranged in order on the microarray such that members of the same group are located in proximity to one another. The probe molecules may, for instance, be arranged in order in a grid pattern on the substrate, such that each row of the grid represents a group sharing the same or similar characteristics, for example, being derived from the same histone protein (e.g. H1), or being derived from the same histone modification type. By way of example, a first row of the grid may comprise probe molecules adapted to hybridise with an antibody having specificity for a certain histone protein, isoform, variant or modification thereof, and a second row of the grid may comprise probe molecules adapted to hybridise with an antibody having specificity for a different histone protein, isoform, variant, or modification thereof.

In a particularly preferred embodiment, the microarrays comprise a population of probe molecules comprising at least two copies of a grid of 196 spots, such that the glass slide has at least two independent copies of any pattern of antibody binding. The grids may comprise a mixture of peptides derived from one or more histones (for example histone H2A and H2B), and adjacent spots in either rows or columns are preferably the same peptide probe molecule. Advantageously, both the 'repeat spotting' of adjacent peptides and the duplicate grid arrangement gives confidence to users that the pattern of antibody binding is clear and reproducible.

A position of the substrate, which is functionalised with a probe molecule is referred to as a sensor site, a probe site, or a spot site. It should be appreciated that it is the detection of hybridisation between an antibody and a probe molecule at a particular sensor site on the microarray substrate that is important, as well as the detection of the presence or absence of the antibody *per se.* Hence, it is preferred that the method according to the invention comprises detecting the location of a hybrid formed between the antibody and a probe molecule in step (ii) of the method.

Hence, the position of the probe molecule on the substrate is such that individual sites may be defined by patterned metallic layers, such as gold, with populations of the probe molecule being immobilised thereto, for example, by using self-assembled monolayer chemistries that may or may not involve spotting technologies for positioning control. The substrate may therefore comprise at least one population of probe molecules immobilised thereto. However, populations of different probe molecules on the substrate may be immobilised at separate sites thereon, with each site supporting a different population of probe molecules. Each population may typically comprise a single probe type. However, the same probe may be repeated at different sites on the sensor surface in order to increase the analytical confidence of the method by using this replicate approach. In addition, it may be preferred to mix different probe molecule types upon the same substrate in order to reduce surface area of the substrate while also still enabling complete discrimination between different antibodies.

Preferably, the substrate support comprises at least two or more probe sites. For sensitive applications, a small number of total probe sites is preferred. Preferably, the area of the substrate support is as small as possible with individual probe sites being about 10-1000µm in diameter, more preferably about 50-750µm in diameter, even more preferably, about 200-500µm in diameter. Preferably, each probe site is about 300-400µm in diameter in diameter/width or smaller, and spaced apart from each other by a small distance. Preferably, the distance between each probe site (i.e. the spacing distance) is as small as possible but sufficiently large to avoid cross-contamination of sequences between each site, and is dependent on the spatial control of the probe molecule deposition method. Preferably, the probe site spacing is about 500µm or less, more preferably about 300µm or less, and most preferably, about 200µm or less.

The microarray may further comprise a control, for example, a biotin-conjugated antibody (e.g. rabbit antibody) functionalised to the substrate support. The control may be spotted at each of the four corners of the microarray substrate. This enables the operator to automatically determine the boundaries of the microarray when using a standard anti-rabbit 'secondary antibody' to detect the test antibody distribution as described in the Examples.

Preferably, the microarray is exposed to a blocking agent prior to step (i) of the method. An example of a suitable blocking agent includes a 10% solution of Bovine Serum Albumin, dissolved in PBS. Then, step (i) of the method is carried out which comprises contacting the histone microarray with at least one antibody (i.e. referred to herein as a 'target'), which is preferably in solution. The array is preferably incubated with a solution containing the antibody to be screened (which may have been typically raised in rabbit) at an appropriate dilution in PBS, 0.1% Tween® for about 1 hour.

Preferably, the method comprises at least one, and more preferably at least two, washing steps. For example, the array may be washed for about 5 minutes in 1M NaCl, 1 x PBS, 0.1% Tween®, and then washed again for 3 x 15 minutes in PBS, 0.1% Tween®. After the washing steps, the detection step (ii) of the method is carried out. Step (ii) may comprise contacting the microarray with a detection antibody, which has specificity for the target antibody, and which may be detected by suitable means. For example, the detection antibody may be a commercially available anti-rabbit antibody, or some other appropriate 'secondary antibody'. Preferably, the detection antibody comprises a label, for example, horseradish peroxidase (HRP). However, it is preferred that the antibody comprises a fluorescent tag, such as *Li-cor* fluorescent tag. Distribution of target antibody bound to the microarray may be detected by detecting for the second antibody bound thereto, for example, by commercial scanner technology (Li-cor).

Following the detection step (ii), the method may comprise a step of screening, identifying, isolating, and/or characterising the detected histone antibody. Hence, the invention provides a method for screening histone-specific, and modified histone specific antibodies, including histone variants and isoforms, i.e. a histone antibody screen. Advantageously, the method according to the invention may therefore be used to identify or discriminate whether the target antibody has specificity for:- (i) a type of histone modification *per se* (e.g. phosphorylation, acetylation, ubiquitination, or methylation etc); (ii) a type of histone modification on a type of amino acid residue (e.g. phosphorylation of serine residues etc); or (iii) a type of histone modification on a specific amino acid residue in the histone protein (e.g. phosphorylation of serine 10 on H3).

In another embodiment, the method of the inventionmay be used to detect or discriminate at least two or more different antibodies. Hence, step (i) of the method may comprise contacting the microarray with at least two different target antibodies, which may or may not be in the same solution. The inventors believe it will be possible to discriminate between each target antibody and their specificities for different histone modifications *per se,* or type of modification as summarised above, by using antibodies raised in different species, and subsequently using different detection ('secondary') antibodies each having specificity for different species antibodies, and each comprising a different label. By way of example, one detection antibody may comprise a Li-cor CR800 tag and another detection antibody may comprise a horse radish peroxidase tag. Hence, the user may detect the different labels on each detection antibody using suitable detectors, and thereby detect and discriminate the binding of the different target antibodies on the microarray.

Hence, it will be appreciated from the foregoing that the microarray according to the first aspect has significant utility for detecting and characterising histone specific antibodies. Furthermore, as mentioned herein, histones are chemically modified by marks or modifications, such as phosphorylation, methylation, acetylation, or ubiquitination, with each of these modifications being carried out by a histone-specific enzyme. For example, histone acetyl transferase is the enzyme which catalyses the acetylation of a histone, for example, on lysine 9 of H3. It will also be appreciated that there is a significant need for researchers to be able to characterise the reactivity and specificity of these histone-specific enzymes. Hence, advantageously, the inventors envisage that the microarray according to the first aspect may also be used to detect or characterise the specificity of such histone-specific enzymes.

Hence, according to a further aspect of the present invention, there is provided a method of detecting a histone-specific protein, the method comprising:-
(i) contacting a histone-specific protein target molecule with a histone microarray according to the first aspect of the invention; and
(ii) detecting the presence or absence of a histone-specific protein, either binding to, or enzymatically acting on, a probe molecule.

By the term "histone-specific protein", we mean an amino acid sequence having specificity with or for a histone protein, i.e. a histone specific binding protein. For example, an antibody would fall within this definition. However, preferably, the histone-specific protein to be detected, and which is contacted with the microarray comprises a histone specific enzyme. The skilled technician will appreciate the various types of histone-specific enzymes that exist. It is therefore preferred that the histone-specific enzyme is independently selected from a group of enymes including:- histone acetyl-transferases and de-acetylases; histone methyl transferases and demethylases; histone E3 ubiquitin ligases; and histone kinases and phosphatises; or any combination thereof. Example 3 presented below demonstrates the use of a histone microarray of the invention to analyse the effects of histone modifications on the enzyme activity of histone modifying enzymes.

Preferred histone modifications, which may be carried out by the enzyme include histone phosphorylation, methylation, acetylation, or ubiquitination, or the removal of these modifications. Especially preferred histone modifications, which may be carried out by the enzyme include: methylation of arginine or lysine; phosphorylation of threonine or serine; acetylation of lysine; or ubiquitination of lysine.

It will be appreciated that in order for the histone specific enzyme to catalyse the modification reaction on the probe molecule, e.g. the methylation, acetylation or ubiquitination of lysine, or phosphorylation of serine, the method preferably comprises providing the enzyme with a suitable substrate molecule, for example, S-adenosyl methionine, acetyl-CoA, or ATP. Preferably, the substrate molecule is labelled, for example, with a radiolabel (e.g. P³², S³⁵ and H³) or with a suitable fluorophore. The label enables an operator of the microarray to detect in step (ii) of the method whether or not a histone modification reaction has been carried out by the enzyme, and if so, on which probe molecule.

More preferably, the deposition, or removal of modifications from substrate molecules by the enzyme may be detected by the use of antibodies specific to defined modifications at specific histone residues. This will permit the sensitive detection of both enzyme catalysed deposition, or removal of modifications at defined residues, and by the comparison of the extent of activity at different histone peptides, permit the identification of which sequences and modifications contribute to enzyme activity.

The method of this aspect of the invention can also be used to detect the binding of a histone specific binding protein to amino acid sequences derived from histones. In this embodiment of the invention, the microarray of the invention is exposed to a histone specific protein and the presence, absence, amount or specificity of binding of that binding protein to a probe molecule is measured. The skilled technician will appreciate the various types of histone-specific binding protein that exist. It is therefore preferred that the histone-specific binding protein is independently selected from a group of binding protein including:- HP1; Histone H1; HMGD; polycomb proteins.

Such an embodiment of the invention can be used to determine the effects of histone modifications on the binding activity of a histone-specific binding protein. For example, the effect of changes to histone modifications at or near to the position of histone binding of the binding protein can be investigated using this method of the invention. Furthermore, the embodiment of this aspect of the invention may have application in measuring the activity of a histone-specific binding protein in different reaction conditions. For example, the binding activity of a histone-specific binding protein (e.g. HP1) to a histone microarray of the invention when the reaction is performed in the presence of nuclear extracts can be determined. This application of the method of the invention may allow the measurement of histone-specific binding protein activity when that protein is not subjected to time-consuming purification. Moreover, such an assay may be used to identify or study the role(s) of additional or unidentified biochemical activities that may be present in nuclear extracts.

The binding of a histone-specific binding protein to one or more probe molecules on the histone microarray of the invention can be assessed by the use of antibodies specific to the histone-specific binding protein, as would be appreciated by the skilled person.

The at least two probe molecules on the substrate support may comprise a sequence derived from histone H1 protein, histone H2A protein, histone H2B protein, histone 3 protein, and/or histone 4 protein, or an isoform, or a variant, or a modification of any of such histone proteins. Any of these probe molecules may be modified by a catalytic reaction with a histone-specific modifying enzyme to either deposit, or remove a histone modification, e.g. acetyl lysine, phosphothreonine, phosphoserine, ubiquitin lysine, etc.

For a better understanding of the invention, and to show how embodiments of the same may be carried into effect, reference will now be made, by way of example, to the accompanying diagrammatic drawings, in which:-
Figure 1 is a schematic representation of histones: H3, H4, H2A, and H2B, and their known modifications, including acetylation (Ac), methylation (Me), phosphorylation (P), and ubiquitination (U) as indicated, (taken from promotional literature from AbCam Ltd, UK);
Figure 2 shows a schematic representation of a 'test' peptide probe molecule and a glass substrate surface, which together form a microarray in accordance with the invention. The Figures shows the components before the peptide probe has been bound to the substrate surface;
Figure 3 shows a schematic representation of the peptide probe having been attached to the substrate surface thereby forming a microarray in accordance with the invention;
Figure 4 shows a series of peptides used as probes on an array in accordance with the present invention for detecting histone H3 and modifications thereof;
Figure 5 shows a series of peptides used as probes on an array in accordance with the present invention for detecting histone H4 and modifications thereof;
Figure 6 shows a series of peptides used as probes on an array in accordance with the present invention for detecting histone H2A and modifications thereof;
Figure 7 shows a series of peptides used as probes on an array in accordance with the present invention for detecting histone H2B and modifications thereof;
Figure 8 shows results of a screening assay for antibody (1), which had specificity for histone acetylation at histone H4 lysine 5;
Figure 9 shows results of a screening assay for antibody (2), which had specificity for histone acetylation at histone H4 lysine 16;
Figure 10 shows results of a screening assay for antibody (3), which had specificity for histone acetylation at histone H4 lysine 8;
Figure 11 shows results of a screening assay for antibody having specificity for histone H3 serine 10 phosphorylation (H3ser10phosp);
Figure 12 shows results of a screening assay for antibody having specificity for histone H3 lysine 4 tri-methylation (H3K4me3); and
Figure 13 shows results of a screening assay for antibody having specificity for histone H3 lysine 4 di-methylation (H2K4me2).
Figure 14: Histone tails are substrates for a number of opposing families of histone modifying enzymes, which either deposit or remove epigenetic 'marks', and include histone deacetylases (HDACs), histone acetyl-transferases (HATs), histone methyl-transferases (HMTs) and histone demethylases (HDMs).
Figure 15. SET 7/9 dependent methylation of micro-array bound histone peptides. Microarray slides containing duplicate arrays (dashed boxes) of 48 discrete histone H3 peptides containing a wide range of histone modifications at defined residues. These were incubated with the methyl-transferase enzyme SET7/9 (Bottom panel) or a buffer containing no enzyme (Control, top panel), and subsequently probed with an anti-H3K4me1 antibody.

### Experimental

The inventors investigated the specificity of antibodies for various histone proteins and histone modifications, as illustrated in Figure 1, and developed a microarray system for screening and/or identifying (i) histone-specific antibodies; and (ii) antibodies against specific histone modifications.

The array consisted of a support surface or substrate, which was 'functionalised' or coated with a series of peptide sequences or epitopes (i.e. referred to herein as 'probes'), which corresponded to the various histone proteins (H3, H4, H2A and H2B) and their modifications, as illustrated in Figure 1. The micro-array was then exposed to a solution containing an antibody (i.e. referred to herein as a 'target') in order to identify or discriminate whether the target antibody had specificity generally for:- (i) a type of histone modification *per se* (e.g. phosphorylation, acetylation, or methylation etc); (ii) a type of histone modification on a type of amino acid residue (e.g. phosphorylation of serine residues); or (iii) a type of histone modification on a specific amino acid residue in the histone protein (e.g. phosphorylation of serine 10 on H3).

### Materials and Methods

### (A) Peptide synthesis

All of the 'test' 'peptides or probes, which corresponded to a histone protein or modification thereof, and which were used to coat the substrate surface were made by solid phase synthesis, using a conventional Fmoc protection strategy incorporating a synthesis resin, as reviewed by Chan and White (W. C. Chan., P. D. White., (2000) - "Fmoc Solid Phase Peptide Synthesis, A Practical Approach", Oxford University Press), although any convenient peptide synthesis device may be used for their production.

Most of the peptide probes were about 21 amino acids long and, as shown in Figure 2, each peptide incorporates a Biotin residue, which is used to assist attachment of the peptide to the substrate surface. The biotin residue of each test peptide was separated from the peptide sequence corresponding to the histone or histone modification by means of two contiguous aminohexanoic acid (H₂N(CH₂)₅CO₂H) spacer molecules. These spacers were included to move or space the test peptide sequence away from the immobilised portion of the molecule. The peptides were cleaved off the synthesis resin using standard acid treatment and appropriate scavenger molecules, as described in Chan and White.

### (B) Peptide probes

Referring to Figure 4, there is provided a list of the sequences for probes for functionalising the substrate, which sequences correspond to human histone 3 (H3) and modifications thereof. The key in the Figure shows the various modifications made to histone H3. In total, 103 peptide sequences were used as probes for histone H3.

Referring to Figure 5, there are listed peptide sequences used as probes for human histone 4 (H4) and modifications thereof. In total, 44 sequences were used as probes for histone H4.

Referring to Figure 6, there are listed peptide sequences corresponding to human histone 2A (H2A) and modifications thereof. In total, 20 sequences were used as probes for histone H2A.

Referring to Figure 7, there are listed peptides corresponding to human histone 2B (H2B) and modifications thereof. In total, 39 sequences were used as epitopes for histone H2B.

Hence, the peptide sequences shown in Figures 4-7 therefore constitute a comprehensive and systematic array of probes for the four human histone proteins, H2A, H2B H3 and H4. However, it will be appreciated that Figures 4-7 is not meant to form an exhaustive list given the differences in amino acid sequence in different histone variants, isoforms and species. As such, the lists of peptide probes given provides an example of the concept of generating a comprehensive/systematic probe array to define histone antibody specificity.

### (C) Peptide micro array manufacture

As shown in Figures 2 and 3, the peptide probe sequences (21 amino-acid long) were immobilised on glass microscope slides, which acted as the substrate surface. Hence, the microarray consisted of the glass substrate coated with a series of test peptide probes. As shown in Figures 2 and 3, the slides were pre-coated with a layer of Streptavidin, a protein, which irreversibly binds the compound Biotin present on the test peptide. All of the peptides include a Biotin molecule attached at the amino-teminus, which forms a bond upon contact with the streptavidin layer on the substrate, thereby forming the micro-array: The array was made by spotting a solution of each of the test peptide probes onto the treated microscope slides in an appropriate grid-like formation, with each test peptide probe being bound to the coated slide substrate as a monolayer.

### (D) Array printing

Any device capable of spotting the test peptides onto the substrate slides would be suitable for printing the arrays. The arrays had spot sizes of approximately 200 to 500 micrometer diameter, which are large enough to be read by any detector. The synthesised peptides were diluted to an approximate concentration of 5 nanomoles/ml in a solution of 60% acetonitrile, 40% water and 0.05% trifluoracetic acid. It is believed that substantially higher concentrations of peptide probe is likely to smear during the analysis step and create problems of interpretation, hence concentrations of not more than 5 nmole/ml were used. Sufficient peptide probe solution, typically 0.1 - 0.2 microlitre, were used to form a spot approximately 200 to 500 micrometer in diameter in duplicate onto the slides. The duplication was found to be important in improving the confidence of analysing the data.

As a control, a biotin-conjugated rabbit antibody was spotted at each of the four corners of the micro array substrate. This enabled the analyst to automatically determine the boundaries of the array when using a standard anti-rabbit 'secondary antibody' to detect the test antibody distribution. After spotting, the slides were stored in slide containers in an atmosphere of either nitrogen or argon.

### (E) Antibody screening

Antibody screening was essentially the same as for the widely used 'ELISA' or 'Western blotting' protocols, as described in Sambrook, Fritsch & Maniatis (Sambrook Fritsch & Maniatis (1989) - "Molecular Cloning; A Laboratory Manual", Cold Spring Harbor laboratory Press). The inventors performed the screening in sealed bags to minimize the volumes of solutions required in the method of the invention. The peptide microarray was first 'blocked' by incubation for 1 hour in a 10% bovine serum albumin solution (Sigma) in phosphate buffered saline (PBS: sodium phosphate NaCl pH 8). The array was then incubated with a solution containing the antibody to be screened (which was typically raised in rabbit) at an appropriate dilution in PBS, 0.1 % Tween® for 1 hour. The array was then washed for 5 minutes in 1M NaCl, 1 x PBS, 0.1% Tween®, and then washed again for 3 x 15 minutes in PBS, 0.1 % Tween®.

After the washing steps, the array was then incubated with a commercially available anti-rabbit antibody, or some other appropriate 'secondary antibody' (for example, *Li-cor* fluorescently tagged anti-rabbit antibody; IRDye 800 CW Goat anti- Rabbit). This was diluted to an appropriate concentration (1:3000) in 10% bovine serum albumin, in PBS, 0.1% Tween®, incubated for 1 hour, and the slide briefly washed (5 minutes) in PBS, 0.1% Tween®. Finally, the distribution of bound antibody was detected on the microarray substrate by commercial scanner technology (Odyssey System, Li-cor Biotechnology, 4308 Progressive Avenue, PO Box 4000, Lincoln Nebraska, USA 68504-5000).

### Examples

The inventors screened a number of different types of antibody that were raised against either (i) the same histone modification at distinct locations (i.e. modifications at specific histone residues); or (ii) different classes of post-translational histone modification.

### Example 1 - Histone acetylation antibodies

Initial tests used a number of antibodies, which were raised against specific acetylated residues in the context of the histone (H4) amino acid sequence as summarised in Figure 1. Three different types antibody specific for histone acetylation modifications which were examined were:-
(1) Histone H4 lysine 5 acetylation (H4K5ac);
(2) Histone H4 lysine 16 acetylation (H4K16ac); and
(3) Histone H4 lysine 8 acetylation (H4K8ac).

Referring to Figures 8-10, there are shown the results of binding studies of antibodies (1), (2) and (3), respectively, on a microarray substrate. Probe arrays were spotted in grid-like manner forming a series of 10 columns and 5 rows, in duplicate, per glass slide substrate, with a control marker column (M) (i.e. a biotinylated rabbit antibody) at column 11 (i.e. the black circles in key). The identical duplicate spotting can be seen in the Figures with a first set of 11 columns (i.e. columns 1-10 and the M column) on the left hand side, and a second set of 11 columns (i.e. columns 1-10 and the M column) on the right hand side.

Probes spotted on the glass substrate contain biotinylated peptides consisting of the first 24 amino acid residues of the histone H4 sequence (as SEQ ID No. 104 as shown in Figure 5) with acetylated lysines at the following residues:-
Columns:-

| **Column Number** | **Histone Modification Type** |
|---|---|
| 1 | No modifications |
| 2 | K5ac, K12ac |
| 3 | K8ac, K12ac |
| 4 | K12ac |
| 5 | K16ac |
| 6 | K5ac, K8ac, K12ac, K16ac |
| 7 | K8ac, K12ac, K16ac |
| 8 | K5ac, K12ac, K16ac |
| 9 | K5ac, K8ac, K16ac |
| 10 | K5ac, K8ac, K12ac |
| M | Markers (biotin - rabbit antibodies) |

The rows in Figures 8-10 contain a serial dilution of the same peptide probes to assess the detection limit (i.e. 200µg/ml for the uppermost row down to 12.5µg/ml for the lowermost row).

### Results

As shown in Figure 8, for antibody (1), i.e. Histone acetylation at histone H4 K5, binding was strong at spots containing K5ac (i.e. columns 2, 6, 8-10) as expected, but in addition, weaker 'non-specific' binding is also observed at K12ac (2,3 & 7), which was not expected.

As shown in Figure 9, for antibody (2), i.e. Histone acetylation at histone H4 lysine 16 acetylation (H4K16ac), binding was strong at spots containing K16ac (i.e. columns 5, 6, 7, 8, 9) as expected. However, no 'non-specific' was observed for K5ac (Row 2 &10), K8ac (Rows 3 &10) or K12ac (Rows 2 - 4 & 10).

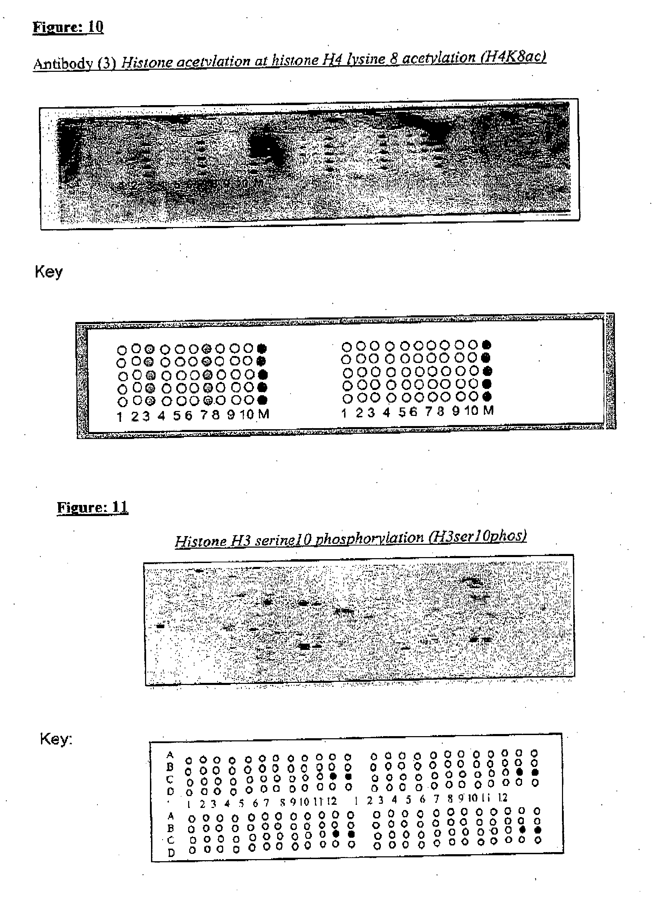

As shown in Figure 10, for antibody (3), i.e. Histone acetylation at histone H4 lysine 8 acetylation (H4K8ac), for spots in columns 3 and 7 there is strong binding indicating that lysine 8 acetylation is recognised. However, interestingly, this binding is blocked when lysine 5 is acetylated (i.e. columns 9 & 10).

### Summary

Hence, the ELISA studies described herein using the microarrays prepared by the inventors have shown that antibody (1) is non-specific, because it also recognises acetylated lysines at lysines 8 and 12, and not just lysine residue 5. However, antibodies (2) and (3) are highly specific for the combination of modification, residue and sequence context. However, the results suggest that antibody (3) is sterically blocked or hindered from binding to it's corresponding probe sequence by modification of adjacent residues.

### Example 2 - Microarrays probing other antibody classes

These arrays are more complex than those described in Example 1, in that they contain four grids of 24 peptides spotted in adjacent pairs, i.e. 12 columns (1-12), i.e. 6 pairs of adjacent peptides x 4 rows (labelled A, B, C, D). Peptides spotted on the substrate were based on histone H3 sequence, with single or multiple modifications. The letters represent standard amino acid code, with the following letters representing modified amino acids.
**Z** phospho threonine
**X** phospho serine
**J** di-methyl lysine
**O** tri methyl lysine
**C** acetyl lysine
**W** mono methyl lysine

| | |
|---|---|
| A1/2 | A R **Z** K Q T A R K S T G G K A P R K Q L A |
| A3/4 | A R **Z O** Q T A R K S T G G K A P R K Q L A |
| A5/6 | A R T **W** Q T A R K S T G G K A P R K Q L A |
| A7/8 | A R T **J** Q T A R K S T G G K A P R K Q L A |
| A9/10 | A R T **O** Q T A R K S T G G K A P R K Q L A |
| A11/12 | A R T **O** Q T A R C S T G G K A P R K Q L A |
| | |
| B1/2 | A R T **O** Q T A R **O** S T G G K A P R K Q L A |
| B3/4 | A R T K Q T A R **C S** T G G K A P R K Q L A |
| B5/6 | A R T K Q T A R C **X** T G G KA P R K Q L A |
| B7/8 | A R T K Q T A R **C** S **Z** G G K A P R K Q L A |
| B9/10 | A R T K Q T A R **C X Z** G G K A P R K Q L A |
| B11/12 | A R T K Q T A R **W** S T G G K A P R K Q L A |
| | |
| C1/2 | A R T K Q T A R **J** S T G G K A P R K Q L A |
| C3/4 | A R T K Q T A R **O** S T G G K A P R K Q L A |
| C5/6 | A R T K Q T A R **O** S **Z** G G K AP R K Q L A |
| C7/8 | A R T K Q T A R **O X T** G G K A P R K Q L A |
| C9/10 | A R T K Q T A R **O X Z** G G K A P R K Q L A |
| C11/12 | A R T K Q T A R K **X** T G G K A P R K Q L A |
| | |
| D1/2 | K A P R K Q L A T K A A R **W** S A P A T G G |
| D3/4 | K A P R K Q L A T K A A R **J** S A P A T G G |
| D5/6 | K A P R K Q L A T K A A R **O** S A P A T G G |
| D7/8 | K A P R K Q L A T K A A R K S A P A T G G |
| D9/10 | K A P R K Q L A T K A A R **O X** A P A T G G |
| D11/12 | K A P R K Q L A T K A A R K S A P A T G G |

Three different types antibody specific for histone acetylation modifications which were examined were:-
(a) Histone H3 serine10 phosphorylation (H3ser10phos);
(b) Histone H3 lysine 4 tri-methylation (H3K4me3); and
(c) Histone H3 di-methyl K4 (H2K4me2).

### (a) Histone H3 serine10 phos-phorylation (H3ser10phos)

As shown in Figure 11, the antibody is highly specific for H3S10phos as spots C11-C12 are bound. However, antibody binding at this site is blocked by adjacent modifications at lysine 9, such as acetylation (spots B5 / B6) or tri-methylation (spots C7 /C8).

### (b) Histone H3 lysine 4 tri-methylation (H3K4me3)

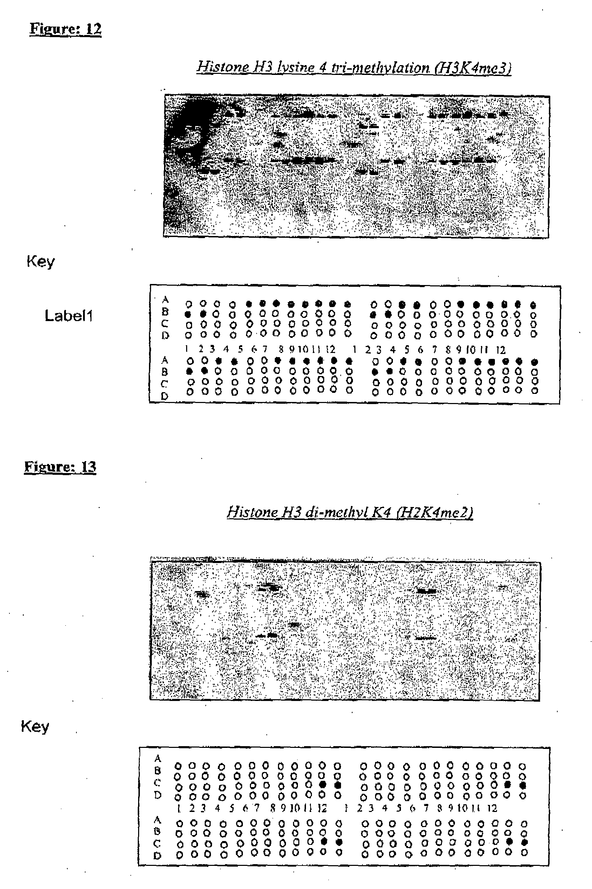

As shown in Figure 12, the antibody is specific to both the modified peptide it was raised with (H3K4me3), but also binds a closely related epitope (H3K4me2). The antibody binds tri-methylation at lysine 4 (B1 / B2), and di-methylation at this residue (A7/A8), but does not bind mono-methyl marks at this residue (A5 /A6) or tri-methyl marks at another lysine residue in histone H3 (lysine 9 - C3 /C4). Antibody binding is occluded by modification at an adjacent site (phosphorylation at threonine 3 - see A3/A4), but not at more distant sites (acetylation at lysine 9 - see A11/A12)

### (c) Histone H3 di-methyl K4 (H2K4me2)

As shown in Figure 13, the antibody is highly specific as it binds di-methyl K4 (A7 /A8), but not mono- (A5 / A6) or tri methylated marks (A9 / A10) at this residue.

### Summary

In summary, the inventors synthesised a microarray containing two duplicate copies of about 206 peptides based on the histone proteins. These peptides consisted of 21 amino acid peptides from a single histone sequence containing:- (i) no modifications; (ii) one modification; or (ii) a number of modifications reflecting what is observed in cells. The micro-array was exposed to a solution containing an antibody (i.e. referred to herein as a 'target') in order to identify or discriminate whether the target antibody had specificity generally for:- (i) a type of histone modification *per se* (e.g. phosphorylation, acetylation, or methylation etc); (ii) a type of histone modification on a type of amino acid residue (e.g. phosphorylation of serine residues); or (iii) a type of histone modification on a specific amino acid residue in the histone protein (e.g. phosphorylation of serine 10 on H3).

Thus, the invention provides a systematic and comprehensive array of many highly related probes. Antibody binding (or non-binding) thereby allows the comprehensive, and accurate definition of the antibody's specificity. It will be appreciated that the microarray and the method enables screening of a number of classes of post-translational modifications present on histones, histone isoforms and histone variant proteins in all species, and not solely human.

### Example 3 - Microarrays assays for modification enzyme and protein binding specificity.

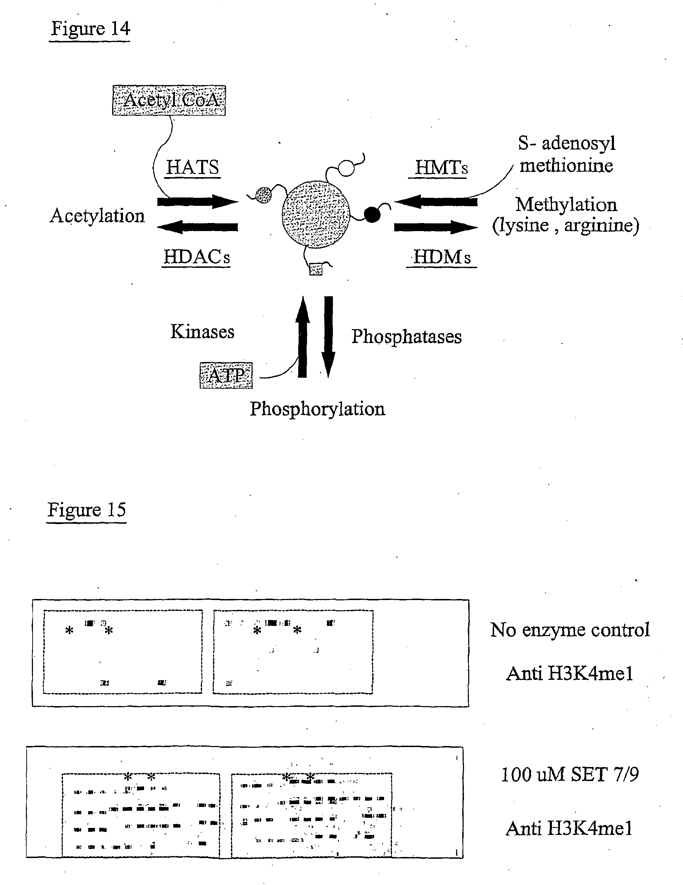

Epigenetic regulation is determined by a number of different classes of proteins which interact with the histone tails. The largest class are the broad range of histone modifying enzymes that regulate the targeting and turnover of the histone marks, via catalysing their deposition (or removal) (Fig. 14). These enzymes play a central role in transcriptional regulation, via the subsequent recruitment of functional 'effector' proteins, and are of wide biological and clinical interest, particularly as the mis-regulation of histone acetyl-transferases and methyl-transferases has been linked to several forms of leukaemia. A large number of 'effector' proteins also bind to histone targets, including chromatin remodelling complexes, linker histones, and proteins involved in stabilizing heterochromatin (HP1).

Such enzymes are termed "histone modifying enzymes". These are well studied for histone acetylation where a large number of histone acetyl-transferases (HATs), and histone deacetylases (HDACs) have been identified. Similarly, histone phosphorylation, and methylation is regulated by different classes of enzymes, though many of the histone lysine demethylases (HDMs) remain unidentified.

A large number of effector proteins are now known to bind, or modulate their activity in response to distinct histone modifications. This is consistent with recent findings that both a structural chromatin binding protein, HP1, and a histone modifying enzyme LSD1 are 'regulated', or show altered binding affinity in response to several marks on adjacent histone residues. Thus, analysis of chromatin binding and modifying proteins should ideally examine a wide range of modified histone peptides as potential interaction partners or substrates. However the practical realities of experimentally testing the myriad combinations of modifications potentially present on a single histone tail represents an expensive and time consuming process.

We therefore explored whether histone peptide microarrays can be used to analyse the effects of histone modification on (1) the enzyme activity of histone modifying enzymes and (2) the histone binding of a number of chromatin binding proteins. Our current data (Fig. 15) suggests that these approaches are experimentally viable.

### Histone peptide naicroarray-based methylation assays (SET 7/9)

Methylation assays were performed using the recombinant human histone methyl-transferase SET 7/9 under standard methylation conditions. Assays on the microarrays of the invention were performed under coverslips, enabling small volumes of reagent usage per assay (70 µl). Enzyme-mediated methylation is detected by a histone methyl specific antibody (i.e. anti-histone H3 mono-methyl K4) by standard Western blotting conditions.

The data presented in Figure 15 shows the distribution of this defined methyl mark on the microarray under control incubations (12 hrs, 30° C methylation buffer), and in the presence of a high concentration (100uM, 70ul) of SET 7/9 enzyme. The antibody detects spots that contain this epitope (*) under control conditions, but also detects a large number of newly created additional sites containing this epitope after HMTase incubation, indicating that specific enzymatic methyl transferase activity occurs under these conditions. Importantly, reproducible patterns of modification are generated on the different peptides (the dashed boxes contain duplicate arrays of 48 epitope spots) allowing users to analyse the effects of defined modifications on methyl transfer by the enzyme.

The data presented indicate that glass-bound histone peptides are both accessible to, and sufficiently concentrated for efficient enzyme action, and that enzyme products (i.e. methylated lysine K4) can be sufficiently abundant to be detected by specific antibodies. As such, this allows the analysis of the impact of a wide range of histone modifications on enzyme catalysis, and opens up a number of experimental avenues.

### Binding studies.

The ability to examine the effect of defined histone modifications on the binding of histone or chromatin binding proteins is also of potentially wide interest. The data presented on Fig 15 suggest that glass bound histone peptides are available for binding. This approach should be valuable for examining all high affinity histone binding proteins if high specificity antibodies against these proteins are available. The technology should be valuable for studying protein binding both in the context of highly purified proteins, and in the context of nuclear extracts (i.e. from Hela, or other tissue culture lines). This would be particularly powerful, as it would facilitate experiments studying putative histone binding proteins (or experimental mutants), in their native protein complexes, without time-consuming purification.

### Summary

The inventors have successfully demonstrated that a histone microarray of the invention can be used to detect the enzymic activity of a histone modifying enzyme, illustrating the application of the microarrays as a research tools for the investigation of epigenetic regulation. Further uses of the microarray are envisaged, including the investigation of the effect of histone modifications on the binding activity of histone or chromatin binding proteins.

## Claims

1. A histone microarray for characterising the specificity of histone-specific proteins, said microarray comprising a substrate support functionalised with a plurality of probe molecules, each probe molecule comprising a peptide sequence, or a derivative or analogue thereof, derived from a histone protein, or isoform, or variant thereof, or one of a series of post-translational modifications thereof.

2. The histone microarray of claim 1, wherein at least one sequence comprises one or more post-translational modifications, and at least one other sequence comprises no post-translational modifications.

3. The histone microarray of claim 1 or claim 2 wherein at least one of the probe molecules is adapted to bind to a histone immunoglobulin target molecule.

4. The histone microarray of any one of claims 1 to 3, wherein the post-translational modifications are independently selected from a group consisting of phosphorylation; methylation; acetylation; and ubiquitination.

5. The histone microarray of any one of claims 1 to 4 wherein the probe molecules comprise sequence derived from one or more histone isoforms independently selected from a group of histone isoforms consisting of: H1.0; H1.1; H1.2; H1.3; H1.4; H1.5; H2A.1; H2A.2; H2A.3; H2B.a; H2B.b; H2B.c, H2A.d, H2A.e, H2A.f; H3.1; H3.2; H3.3; H4; and their related homologues in other species.

6. The histone microarray of any one of claims 1 to 5 wherein the probe molecules comprise sequence derived from one or more histone variants independently selected from a group of histone variants consisting of: H2A-X; H2A-Z; macro-H2A, H2A-Bbd, CENP-A; and their related homologues in other species.

7. The histone microarray of any one of claims 4 to 6, wherein the post-translational modifications are independently selected from:- methylation of arginine or lysine; phosphorylation of threonine or serine; acetylation of lysine; or ubiquitination of lysine.

8. The histone microarray of any one of the previous claims wherein said probe molecules comprise a sequence of at least 10 amino acids and/or a sequence of less than 100 amino acids.

9. The histone microarray of any one of the previous claims wherein the probe molecules are derived from different histone proteins, or isoforms, or variants thereof or the probe molecules are derived from the same histone protein, or isoform, or variant thereof.

10. The histone microarray of claim 9 in which the probe molecules are derived from the same histone protein, or isoform, or variant thereof, comprising at least two probe molecules comprising sequence derived from histone H3 protein, or isoform, or variant thereof.

11. The histone microarray of claim 10 comprising at least two probe molecules comprising a sequence independently selected from a group of sequences identified as SEQ ID Nos 2 to 40; 42 to 69; 71 to 84; 86 to 88; 90 to 92; 94 to 99; 101 to 103, or any combination thereof.

12. The histone microarray of claim 9 comprising at least two probe molecules comprising sequence derived from histone H4 protein, or isoform, or variant thereof.

13. The histone microarray of claim 12 comprising at least two probe molecules comprising a sequence independently selected from a group of sequences identified as SEQ ID No. 105 to 122; 124 to 130; 132; 133; 135; 137 to 144; 146; 147, or any combination thereof.

14. The histone microarray of claim 9 comprising at least two probe molecules comprising sequence derived from histone H2A protein, or isoform, or variant thereof.

15. The histone microarray of claim 14 comprising at least two probe molecules comprising a sequence independently selected from a group of sequences identified as SEQ ID No. 149 to 157; 159; 161 to 163; 165 to 167, or any combination thereof.

16. The histone microarray of claim 9 comprising at least two probe molecules comprising sequence derived from histone H2B protein, or isoform, or variant thereof.

17. The histone microarray of claim 16 comprising at least two probe molecules comprising a sequence independently selected from a group of sequences identified as SEQ ID No. 169 to 177; 179 to 187; 189 to 193; 195 to 197; 199; 201 to 206, or any combination thereof.

18. The histone microarray of claim 9 in which the probe molecules are derived from different histone proteins, or isoforms, or variants thereof, comprising at least two probe molecules comprising sequence derived from histone H2A protein and histone 2B protein, or isoform, or variant thereof.

19. The histone microarray of claim 18 comprising at least two probe molecules comprising a sequence independently selected from a group of sequences identified as SEQ ID no. 149 to 157; 159; 161 to 163; 165 to 167; 169 to 177; 179 to 187; 189 to 193; 195 to 197; 199; 201 to 206, or any combination thereof.

20. The histone microarray of claim 9 comprising at least two probe molecules comprising a sequence derived from histone H1 protein, or isoform, or variant thereof.

21. The histone microarray of claim 9 comprising at least two probe molecules comprising a sequence independently selected from a group of sequences identified as SEQ ID No. 2 to 40; 42 to 69; 71 to 84; 86 to 88; 90 to 92; 94 to 99; 101 to 103; 105 to 122; 124 to 130; 132; 133; 135; 137 to 144; 146; 147; 149 to 157; 159; 161 to 163; 165 to 167; 169 to 177; 179 to 187; 189 to 193; 195 to 197; 199; 201 to 206, or any combination thereof.

22. The histone microarray of any one of the previous claims wherein the probe molecule comprises spacing means adapted to distance the sequence derived from a histone protein, isoform, or variant, or one of a series of post-translational modifications thereof, away from the substrate support when bound thereto, the spacing means preferably comprising at least one molecule of aminohexamoic acid (H₂N(CH₂)₅CO₂H).

23. A method of detecting a histone-specific protein, the method comprising:-
(i) contacting a histone-specific protein target molecule with a histone microarray according to any one of claims 1 to 22; and
(ii) detecting the presence or absence of a histone-specific protein, either binding to, or enzymatically acting on, a probe molecule.

24. The method of claim 23 wherein the histone-specific protein is a histone antibody.

25. The method of claim 23 wherein the histone-specific protein target molecule is a histone-specific enzyme independently selected from a group of enzymes consisting of: histone acetyl-transferases and de-acetylases; histone methyl transferases and demethylases; histone E3 ubiquitin ligases; and histone kinases and phosphatises; or any combination thereof.

26. The method of claim 23 wherein the histone-specific protein target molecule is a histone-specific binding protein independently selected from a group of binding proteins consisting of:- HP1; Histone H1; HMGD; polycomb proteins.

## Patentansprüche

1. Histon-Mikroarray zur Charakterisierung der Spezifität histonspezifischer Proteine, wobei das Mikroarray ein mit mehreren Sondenmolekülen funktionalisiertes Trägersubstrat umfasst, wobei jedes Sondenmoleküle eine Peptidsequenz, oder ein Derivat oder Analog davon, abgeleitet von einem Histonprotein, oder einer Isoform, oder einer Variante davon, oder von einer aus einer Reihe posttranslationaler Modifikationen davon, umfasst.

2. Histon-Mikroarray nach Anspruch 1, wobei zumindest eine Sequenz eine oder mehrere posttranslationale Modifikationen umfasst, und zumindest eine andere Sequenz keine posttranslationalen Modifikationen umfasst.

3. Histon-Mikroarray nach Anspruch 1 oder Anspruch 2, wobei zumindest eines der Sondenmoleküle dazu geeignet ist, an ein Histon-Immunglobulin-Zielmolekül zu binden.

4. Histon-Mikroarray nach einem der Ansprüche 1 bis 3, wobei die posttranslationalen Modifikationen unabhängig aus einer Gruppe ausgewählt sind, die aus Phosphorylierung; Methylierung; Acetylierung; und Ubiquitinierung besteht.

5. Histon-Milwoamay nach einem der Ansprüche 1 bis 4, wobei die Sondenmoleküle eine Sequenz umfassen, abgeleitet von einer oder mehreren Histonisoformen, unabhängig ausgewählt aus einer Gruppe von Histonisoformen, bestehend aus: H1.0; H1.1; H1.2; H1.3; H1.4; H1.5; H2A.1; H2A.2; H2A.3; H2B.a; H2B.b; H2B.c; H2A.d; H2A.e; H2A.f; H3.1; H3.2; H3.3; H4; und deren verwandten Homologen in anderen Spezies.

6. Histon-Mikroarray nach einem der Ansprüche 1 bis 5, wobei die Sondenmoleküle eine Sequenz umfassen, abgeleitet von einer oder mehreren Histonvarianten, unabhängig ausgewählt aus einer Gruppe von Histonvarianten, bestehend aus: H2A-X; H2A-Z; Makro-H2A, H2A-Bbd, CENP-A; und deren verwandten Homologen in anderen Spezies.

7. Histon-Mikroarray nach einem der Ansprüche 4 bis 6, wobei die posttranslationalen Modifikationen unabhängig ausgewählt sind aus: Methylierung von Arginin oder Lysin; Phosphorylierung von Threonin oder Serin; Acetylierung von Lysin; oder Ubiquitinierung von Lysin.

8. Histon-Mikroarray nach einem der vorstehenden Ansprüche, wobei die Sondenmoleküle eine Sequenz von zumindest 10 Aminosäuren und/oder eine Sequenz von weniger als 100 Aminosäuren umfassen.

9. Histon-Mikroarray nach einem der vorstehenden Ansprüche, wobei die Sondenmoleküle von unterschiedlichen Histonproteinen, oder Isoformen, oder Varianten davon, abgeleitet sind oder die Sondenmolekül von dem gleichen Histonprotein, oder der gleichen Isoform, oder der gleichen Variante davon, abgeleitet sind.

10. Histon-Mikroarray nach Anspruch 9, in dem die Sondenmoleküle abgeleitet sind von dem gleichen Histonprotein, oder der gleichen Isoform, oder der gleichen Variante davon, umfassend zumindest zwei Sondenmoleküle mit von Histon-H3-Protein, oder einer Isoform, oder einer Variante davon, abgeleiteter Sequenz.

11. Histon-Mikroarray nach Anspruch 10, umfassend zumindest zwei Sondenmoleküle mit einer Sequenz, die unabhängig ausgewählt ist aus einer Gruppe von Sequenzen, die identifiziert sind als SEQ ID Nr.: 2 bis 40; 42 bis 69; 71 bis 84; 86 bis 88; 90 bis 92; 94 bis 99; 101 bis 103, oder jeder beliebigen Kombination davon.

12. Histon-Mikroarray nach Anspruch 9, umfassend zumindest zwei Sondenmoleküle mit von Histon-H4-Protein, oder einer Isoform, oder einer Variante davon, abgeleiteter Sequenz.

13. Histon-Mikroarray nach Anspruch 12, umfassend zumindest zwei Sondenmoleküle mit einer Sequenz, die unabhängig ausgewählt ist aus einer Gruppe von Sequenzen, die identifiziert sind als SEQ ID Nr.: 105 bis 122; 124 bis 130; 132; 133; 135; 137 bis 144; 146; 147, oder jeder beliebigen Kombination davon.

14. Histon-Mikroarray nach Anspruch 9, umfassend zumindest zwei Sondenmolekül mit von Histon-H2A-Protein, oder einer Isoform, oder einer Variante davon, abgeleiteter Sequenz.

15. Histon-Mikroarray nach Anspruch 14, umfassend zumindest zwei Sondenmoleküle mit einer Sequenz, die unabhängig ausgewählt ist aus einer Gruppe von Sequenzen, die identifiziert sind als SEQ ID Nr.: 149 bis 157; 159; 161 bis 163; 165 bis 167, oder jeder beliebigen Kombination davon.

16. Histon-Mikroarray nach Anspruch 9, umfassend zumindest zwei Sondenmoleküle mit von Histon-H2B-Protein, oder einer Isoform, oder einer Variante davon, abgeleiteter Sequenz.

17. Histon-Mikroarray nach Anspruch 16, umfassend zumindest zwei Sondenmoleküle mit einer Sequenz, die unabhängig ausgewählt ist aus einer Gruppe von Sequenzen, die identifiziert sind als SEQ ID Nr.: 169 bis 177; 179 bis 187; 189 bis 193; 195 bis 197; 199; 201 bis 206, oder jeder beliebigen Kombination davon.

18. Histon-Mikroarray nach Anspruch 9, in dem die Sondenmoleküle abgeleitet sind von unterschiedlichen Histonproteinen, oder Isoformen, oder Varianten davon, umfassend zumindest zwei Sondenmoleküle mit von Histon-H2A-Protein und Histon-2B-Protein, oder einer Isoform, oder einer Variante davon, abgeleiteter Sequenz.

19. Histon-Mikroarray nach Anspruch 18, umfassend zumindest zwei Sondenmolekül mit einer Sequenz, die unabhängig ausgewählt ist aus einer Gruppe von Sequenzen, die identifiziert sind als SEQ ID Nr.: 149 bis 157; 159; 161 bis 163; 165 bis 167; 169 bis 177; 179 bis 187; 189 bis 193; 195 bis 197; 199; 201 bis 206, oder jeder beliebigen Kombination davon.

20. Histon-Milcroarray nach Anspruch 9, umfassend zumindest zwei Sondenmoleküle mit einer von Histon-H1-Protein, oder einer Isoform, oder einer Variante davon, abgeleiteten Sequenz.

21. Histon-Mikroarray nach Anspruch 9, umfassend zumindest zwei Sondenmoleküle mit einer Sequenz, die unabhängig ausgewählt ist aus einer Gruppe von Sequenzen, die identifiziert sind als SEQ ID Nr.: 2 bis 40; 42 bis 69; 71 bis 84; 86 bis 88; 90 bis 92; 94 bis 99; 101 bis 103; 105 bis 122; 124 bis 130; 132; 133; 135; 137 bis 144; 146; 147; 149 bis 157; 159; 161 bis 163; 165 bis 167; 169 bis 177; 179 bis 187; 189 bis 193; 195 bis 197; 199; 201 bis 206, oder jeder beliebigen Kombination davon.

22. Histon-Mikroarray nach einem der vorstehenden Ansprüche, wobei das Sondenmolekül ein Abstandhaltemittel umfasst, das dazu geeignet ist, die Sequenz, abgeleitet von einem Histonprotein, einer Isoform, oder einer Variante, oder einer aus einer Reihe posttranslationaler Modifikationen davon, vom Trägersubstrat zu beabstanden, wenn sie daran gebunden ist, wobei das Abstandhaltemittel bevorzugt zumindest ein Molekül der 6-Aminohexansäure (H₂N(CH₂)₅CO₂H) umfasst.

23. Verfahren zum Nachweis eines histonspezifischen Proteins, wobei das Verfahren umfasst:
(i) Inkontaktbringen eines Histonspezifischen-Protein-Zielmoleküls mit einem Histon-Mikroarray gemäß einem der Ansprüche 1 bis 22; und
(ii) Nachweisen der Anwesenheit oder Abwesenheit eines histonspezifischen Proteins, das entweder an ein Sondenmolekül bindet, oder enzymatisch auf dieses wirkt.

24. Verfahren nach Anspruch 23, wobei das histonspezifische Protein ein Histon-Antikörper ist.

25. Verfahren nach Anspruch 23, wobei das Histonspezifische-Protein-Zielmolekül ein histonspezifisches Enzym ist, das unabhängig ausgewählt ist aus einer Gruppe von Enzymen, bestehend aus: Histon-Acetyltransferasen und -Deacetylasen; Histon-Methyltransferasen und -Demethylasen; Histon-E3-Ubiquitin-Ligasen; und Histon-Kinasen und -Phosphatasen; oder jeder beliebigen Kombination davon.

26. Verfahren nach Anspruch 23, wobei das Histonspezifische-Protein-Zielmolekül ein histonspezifisch bindendes Protein ist, das unabhängig ausgewählt ist aus einer Gruppe bindender Proteine, bestehend aus: HP1; Histon H1; HMGD; Polycomb-Proteinen.

## Revendications

1. Microréseau d'histone pour la caractérisation de la spécificité de protéines spécifiques à une histone, ledit microréseau comprenant un support de substrat fonctionnalisé avec une pluralité de molécules sondes, chaque molécule sonde comprenant une séquence peptidique, ou un dérivé ou un analogue de celle-ci, dérivée d'une protéine d'histone, ou d'une isoforme, ou d'un variant de celle-ci, ou d'une parmi une série de modifications après traduction de celle-ci.

2. Microréseau d'histone selon la revendication 1, dans lequel au moins une séquence comprend une ou plusieurs modifications après traduction et au moins une autre séquence ne comprend pas de modifications après traduction.

3. Microréseau d'histone selon la revendication 1 ou la revendication 2, dans lequel au moins une des molécules sondes est adaptée pour se lier à une molécule cible d'immunoglobuline d'histone.

4. Microréseau d'histone selon l'une quelconque des revendications 1 à 3, dans lequel les modifications après traduction sont indépendamment choisies dans le groupe constitué de phosphorylation; de méthylation; d'acétylation; et d'ubiquitination.

5. Microréseau d'histone selon l'une quelconque des revendications 1 à 4, dans lequel les molécules sondes comprennent une séquence dérivée d'une ou de plusieurs isoformes d'histone indépendamment choisies dans un groupe d'isoformes d'histone constitué de: H1.0; H1.1; H1.2; H1.3; H1.4; H1.5; H2A.1; H2A.2; H2A.3; H2B.a; H2B.b; H2B.c; H2A.d; H2A.e; H2A.f; H3.1; H3.2; H3.3; H4; et leurs homologues apparentés dans d'autres espèces.

6. Microréseau d'histone selon l'une quelconque des revendications 1 à 5, dans lequel les molécules sondes comprennent une séquence dérivée d'un ou de plusieurs variants d'histone indépendamment choisis dans un groupe de variants d'histone constitué de: H2A-X; H2A-Z; macro-H2A; H2A-Bbd; CENP-A; et leurs homologues apparentés dans d'autres espèces.

7. Microréseau d'histone selon l'une quelconque des revendications 4 à 6, dans lequel les modifications après traduction sont indépendamment choisies parmi: une méthylation d'arginine ou de lysine; une phosphorylation de thréonine ou de sérine; une acétylation de lysine; ou une ubiquitination de lysine.

8. Microréseau d'histone selon l'une quelconque des revendications précédentes, dans lequel lesdites molécules sondes comprennent une séquence d'au moins 10 acides aminés et/ou une séquence de moins de 100 acides aminés.

9. Microréseau d'histone selon l'une quelconque des revendications précédentes, dans lequel les molécules sondes sont dérivées de protéines d'histone, ou d'isoformes, ou de variants de celles-ci différents ou les molécules sondes sont dérivées d'une protéine d'histone, ou d'une isoforme, ou d'un variant de celle-ci identique.

10. Microréseau d'histone selon la revendication 9, dans lequel les molécules sondes sont dérivées d'une protéine d'histone, ou d'une isoforme, ou d'un variant de celle-ci identique, comprenant au moins deux molécules sondes comprenant une séquence dérivée de la protéine d'histone H3, ou d'une isoforme, ou d'un variant de celle-ci.

11. Microréseau d'histone selon la revendication 10, comprenant au moins deux molécules sondes comprenant une séquence indépendamment choisie dans un groupe de séquences identifiées comme SEQ ID Nos 2 à 40; 42 à 69; 71 à 84; 86 à 88; 90 à 92; 94 à 99; 101 à 103, ou toute combinaison de celles-ci.

12. Microréseau d'histone selon la revendication 9, comprenant au moins deux molécules sondes comprenant une séquence dérivée de la protéine d'histone H4, ou d'une isoforme, ou d'un variant de celle-ci.

13. Microréseau d'histone selon la revendication 12, comprenant au moins deux molécules sondes comprenant une séquence indépendamment choisie dans un groupe de séquences identifiées comme SEQ ID No. 105 à 122; 124 à 130; 132; 133; 135; 137 à 144; 146; 147, ou toute combinaison de celles-ci.

14. Microréseau d'histone selon la revendication 9, comprenant au moins deux molécules sondes comprenant une séquence dérivée de la protéine d'histone H2A, ou d'une isoforme, ou d'un variant de celle-ci.

15. Microréseau d'histone selon la revendication 14, comprenant au moins deux molécules sondes comprenant une séquence indépendamment choisie dans un groupe de séquences identifiées comme SEQ ID No. 149 à 157; 159; 161 à 163; 165 à 167, ou toute combinaison de celles-ci.

16. Microréseau d'histone selon la revendication 9, comprenant au moins deux molécules sondes comprenant une séquence dérivée de la protéine d'histone H2B, ou d'une isoforme, ou d'un variant de celle-ci.

17. Microréseau d'histone selon la revendication 16, comprenant au moins deux molécules sondes comprenant une séquence indépendamment choisie dans un groupe de séquences identifiées comme SEQ ID No. 169 à 177; 179 à 187; 189 à 193; 195 à 197; 199; 201 à 206, ou toute combinaison de celles-ci.

18. Microréseau d'histone selon la revendication 9, dans lequel les molécules sondes sont dérivées de protéines d'histone, ou d'isoformes, ou de variants de celles-ci différents, comprenant au moins deux molécules sondes comprenant une séquence dérivée de la protéine d'histone H2A et de la protéine d'histone 2B, ou d'une isoforme, ou d'un variant de celles-ci.

19. Microréseau d'histone selon la revendication 18, comprenant au moins deux molécules sondes comprenant une séquence indépendamment choisie dans un groupe de séquences identifiées comme SEQ ID No. 149 à 157; 159; 161 à 163; 165 à 167; 169 à 177; 179 à 187; 189 à 193; 195 à 197; 199; 201 à 206, ou toute combinaison de celles-ci.

20. Microréseau d'histone selon la revendication 9, comprenant au moins deux molécules sondes comprenant une séquence dérivée de la protéine d'histone H1, ou d'une isoforme, ou d'un variant de celle-ci.

21. Microréseau d'histone selon la revendication 9, comprenant au moins deux molécules sondes comprenant une séquence indépendamment choisie dans un groupe de séquences identifiées comme SEQ ID No. 2 à 40; 42 à 69; 71 à 84; 86 à 88; 90 à 92; 94 à 99; 101 à 103; 105 à 122; 124 à 130; 132; 133; 135; 137 à 144; 146; 147; 149 à 157; 159; 161 à 163; 165 à 167; 169 à 177; 179 à 187; 189 à 193; 195 à 197; 199; 201 à 206, ou toute combinaison de celles-ci.

22. Microréseau d'histone selon l'une quelconque des revendications précédentes, dans lequel la molécule sonde comprend un moyen d'espacement adapté à distancer la séquence dérivée d'une protéine d'histone, d'une isoforme, ou d'un variant, ou d'une parmi une série de modifications après traduction de celle-ci, du support de substrat lorsqu'il est lié à celle-ci, le moyen d'espacement comprenant de préférence au moins une molécule d'acide aminohexamoïque (H₂N(CH₂)₅CO₂H).

23. Méthode pour la détection d'une protéine spécifique à une histone, la méthode comprenant:
(i) la mise en contact d'une molécule cible de protéine spécifique à une histone avec un microréseau d'histone selon l'une quelconque des revendications 1 à 22; et
(ii) la détection de la présence ou de l'absence d'une protéine spécifique à une histone, soit se liant à, soit agissant enzymatiquement sur, une molécule sonde.

24. Méthode selon la revendication 23, dans laquelle la protéine spécifique à une histone est un anticorps d'histone.

25. Méthode selon la revendication 23, dans laquelle la molécule cible de protéine spécifique à une histone est une enzyme spécifique à une histone indépendamment choisie dans un groupe d'enzymes constitué de: histone-acétyltransférases et -dé-acétylases; histone-méthyltransférases et -déméthylases; histone-E3-ubiquitine-ligases; et histone-kinases et -phosphatases; ou toute combinaison de celles-ci.

26. Méthode selon la revendication 23, dans laquelle la molécule cible de protéine spécifique à une histone est une protéine de liaison spécifique à une histone indépendamment choisie dans le groupe de protéines de liaison constitué de: HP1; Histone H1; HMGD; protéines polycomb.
